(19) Europäisches Patentamt
European Patent Office
Office européen des brevets

(11) EP 2 320 922 B1

(12) **EUROPÄISCHE PATENTSCHRIFT**

(45) Veröffentlichungstag und Bekanntmachung des Hinweises auf die Patenterteilung:
**17.10.2012 Patentblatt 2012/42**

(21) Anmeldenummer: **09781684.7**

(22) Anmeldetag: **11.08.2009**

(51) Int Cl.:
***A61K 36/9066*** *(2006.01)* ***A61K 36/258*** *(2006.01)*
***A61K 36/82*** *(2006.01)* ***A61K 36/48*** *(2006.01)*
***A61P 25/28*** *(2006.01)*

(86) Internationale Anmeldenummer:
**PCT/EP2009/060358**

(87) Internationale Veröffentlichungsnummer:
**WO 2010/026021 (11.03.2010 Gazette 2010/10)**

(54) **KOMBINATION VON EXTRAKTEN AUS VERSCHIEDENEN PFLANZEN ZUR VERBESSERUNG DER SYMPTOME VON DEMENZERKRANKUNGEN**

COMBINATION OF EXTRACTS OF VARIOUS PLANTS FOR IMPROVING THE SYMPTOMS OF DEMENTIA DISORDERS

ASSOCIATION D'EXTRAITS DE DIFFÉRENTES PLANTES DESTINÉE À AMÉLIORER LES SYMPTÔMES DES DÉMENCES

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **05.09.2008 EP 08015700**

(43) Veröffentlichungstag der Anmeldung:
**18.05.2011 Patentblatt 2011/20**

(73) Patentinhaber: **Kneipp-Werke Kneipp-Mittel-Zentrale GmbH & Co. KG**
**97082 Würzburg (DE)**

(72) Erfinder: **FRANK, Bruno**
**97271 Kleinrinderfeld (DE)**

(74) Vertreter: **Keller, Günter et al**
**Lederer & Keller**
**Patentanwälte**
**Unsöldstrasse 2**
**80538 München (DE)**

(56) Entgegenhaltungen:
**WO-A-2007/057310 ZA-A- 200 303 674**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 320 922 B1

## Beschreibung

Die vorliegende Erfindung betrifft Kombinationen von Extrakten aus verschiedenen Pflanzen, die immer einen Rotbuschextrakt zusammen mit wenigstens zwei weiteren Extraktenaus einer anderen Pflanze aufweisen. Die Extraktkombination kann zur Vorbeugung und/oder Behandlung von Demenzerkrankungen eingesetzt werden.

Die Kombinationen von Extrakten aus verschiedenen Pflanzen dienen im weitesten Sinn als Lebensmittel, insbesondere als Nahrungsmittelergänzungsprodukte sowie als Medikament, insbesondere zur Behandlung von neurologischen und psychiatrischen Störungen des zentralen Nervensystems. Der Ausdruck pharmazeutisch wirksam umfasst auch solche Wirkungen, die zu einer subjektiven Verbesserung der Befindlichkeit führen, wobei eine arzneimittelrechtliche Zulassung nicht unbedingt erforderlich sein muss.

Der Rotbusch (englisch: Rooibos; lateinisch: Aspalathus linearis) wächst ausschließlich in Südafrika und enthält derzeit als weltweit einzige bekannte Pflanze die besonders stark antioxidativ wirkende Substanz Aspalathin, ein Flavonoid. Zudem enthält der Rotbusch weitere Flavonoide, wie C-Glycosylflavone (unter anderem Orientin, Isoorientin), Flavonol-3-O-Glycoside (unter anderem Quercetin, Quercitrin, Isoquercitrin, Rutin) und Glucoside, insbesondere C-Glykoside und Chalkone wie Nothofagin und Aspalathin.

Unfermentierter "grüner" Rotbusch zeichnet sich durch einen höheren Gehalt an Polyphenolen, insbesondere Aspalathin, sowie einer höheren antioxidativen Aktivität im Vergleich zum fermentierten Produkt aus. Der Effekt der Abnahme der antioxidativen Aktivität durch den Fermentationsprozess ist gleichermaßen beim schwarzen gegenüber dem grünen Tee zu beobachten (Bramati et al., J. Agric. Food Chem. 2003, 51: 7472-7474). Wissenschaftliche Untersuchungen haben gezeigt, dass die antioxidative Aktivität des Rotbuschtees hauptsächlich auf den Aspalathingehalt zurückzuführen ist. Bei der Untersuchung des Fermentationsprozesses von Rotbuschtee wurde festgestellt, dass der Gehalt an Aspalathin und Nothofagin während des Fermentationsprozesses abnimmt (Schulz et al., Eur. Food Res. Technol. 2003, 216: 539-543). Die geringere antioxidative Aktivität von fermentiertem "rotem" Rotbuschtee gegenüber unfermentiertem "grünem" Rotbuschtee kann somit erklärt werden.

Aufgrund der vorstehend genannten gesundheitsfördernden Flavonoide sowie seinem guten Geschmack ist der Rotbuschtee weit verbreitet. Weitere Inhaltsstoffe des Rotbuschtees sind Phenolsäuren, ätherisches Öl, Vitamin C sowie zahlreiche Mineralstoffe, insbesondere Eisen.

Um eine möglichst hohe antioxidative Aktivität zu erzielen, ist ein hoher Gehalt an Aspalathin notwendig. Diesbezüglich offenbart DE 10 2005 004 438 einen Rotbuschextrakt, der im Vergleich zu dem üblichen Aspalathingehalt von 1 bis 3 Gew.-% einen erhöhten Gehalt von mehr als 5 Gew.-%, bei gleichzeitig niedrigem Chlorophyllgehalt von weniger als 0,4 Gew.-%, aufweist. Gemäß DE 10 2005 004 438 wird der Rotbuschextrakt durch Extraktion aus unfermentierter Rotbusch-Rohware unter Verwendung eines Gemischs aus 80 Teilen Ethanol und 20 Teilen Wasser erhalten. Der Rotbuschextrakt mit hohem Aspalathingehalt soll aufgrund der stark antioxidativen, antiirritativen und antimikrobiellen Wirkung insbesondere für die kosmetische Anwendung beispielsweise als Haar-, Haut- oder Mundpflegemittel verwendet werden.

Ein weiteres Flavonoid, das in dem Rotbuschtee enthalten ist, ist Quercetin. Dies kommt mit einem Gehalt von ca. 11 mg/100 g Rotbusch-Rohmaterial vor und wirkt sich z.B. auf die Histaminfreisetzung im menschlichen Körper aus, wodurch allergische Symptome gelindert werden können. Quercetin vermag ebenso die Produktion der Monoaminooxidase zu hemmen, was leichte Depressionen und Einschlafstörungen günstig beeinflusst (Plantextrakt, the nature network, Ausgabe 3 vom 09.11.2005; Plantextrakt GmbH).

Ein Ausgangspunkt der vorliegenden Erfindung war die Suche nach Wirkstoffen zur Behandlung von Krankheiten des zentralen Nervensystems, wie beispielsweise Demenzen, Morbus Parkinson, Depressionen und Schmerzzustände. Diese Krankheiten sind schwer zu therapieren, und die hierbei eingesetzten Medikamente wie beispielsweise Tacrin, Galantamin oder Nefopam weisen ein breites Nebenwirkungsspektrum auf.

Bei Demenzerkrankungen werden Alzheimer-Demenz, zerebrovaskuläre Demenz, und Demenzen aufgrund anderer Ursachen (M. Prick, M. Parkinson, Chorea Huntington, und weitere, eher seltene Ursachen) unterschieden, wobei die Alzheimer-Demenz die häufigste Form darstellt. Die früher strenge Trennung von vaskulärere Demenz und Alzheimer-Demenz wird in jüngster Zeit verlassen.

Nach dem derzeitigen Stand der Erkenntnis sind die Behandlungserfolge bei M. Alzheimer-Demenz mit den derzeit verfügbaren Monosubstanzen unbefriedigend: Bislang wurden Cholinesterase-Inhibitoren für leichte bis mäßige und der NMDA-Rezeptor-Antagonist Memantin für mäßige bis schwere Ausprägungen von Alzheimer-Demenzzuständen von den Europäischen und Nordamerikanischen (USA und Kanada) Behörden zugelassen; in Deutschland ist weiterhin ein Extrakt aus Ginko biloba bei Alzheimer Demenz zugelassen. In den klinischen Studien wurden aber mit den genannten Substanzen, zwar mehr oder weniger signifikante Resultate erzielt, doch befriedigt das Ausmaß der Wirkungen keineswegs, weshalb die Suche nach Verbesserungen des Effekts anhält und auch Kombinationen mit einer jeweils zweiten Substanz mit unterschiedlichem Wirkmechanismus zunehmend untersucht wird.

Kombiniert werden in der Regel Cholinesterasehemmer mit einem Calcium-/NMDA-Antagonisten, aber auch hier ist bislang kein eindeutiger therapeutischer Fortschritt im Sinne höherer Ansprechraten und/oder stärkerer Wirkung

erzielt worden.

Für das Anfangsstadium der Erkrankung ist bislang von keiner Behörde eine Zulassung erteilt worden, da kein positives Nutzen-Risiko-Verhältnis für eine der bislang zugelassenen Substanzen nachgewiesen ist. Es gibt eine Reihe von Substanzen, für die aus epidemiologischen Untersuchungen ein signifikanter Präventionseffekt gegen Alzheimer-Demenz nachgewiesen wurde: Statine, nichtsteroidale Entzündungshemmer und Östrogene.

Ätiopathologisch werden bei M. Alzheimer-Demenz in etwa 5-10 % aller Fälle genetische Ursachen angenommen, für die übrigen 90 - 95% werden eine ganze Reihe von pathophysiologischen Ursachen diskutiert. Die in jedem Fall beobachtbare Schädigung der Neurone kann eine Vielzahl von Ursachen haben: (i) eine gestörte zelluläre Calcium-Homöostase, (ii) vermehrt gebildete freie Sauerstoffradikale, (iii) Ansammlungen von β-Amyloid, (iv) Mangel an Wachstumsfaktoren, (v) entzündliche Prozesse, (vi) Induktoren des programmierten Zelltodes (Apoptose), (vii) Störung der Transmittersysteme der cholinergen, dopaminergen oder glutamatergen Signalwege, sowie (viii) sekundäre Folgen von Infektionen mit *Chlamydia pneumoniae* und *Herpes simplex* bzw. Cytomegalie-Virus. Es ist bislang nicht geklärt, welche dieser Prozesse auf welche Weise miteinander zusammenhängen und welche primäre oder sekundäre Prozesse in Bezug auf den Krankheitsverlauf sind, jedoch ist aufgrund der bisherigen klinischen Studien klar, dass ein medikamentöser Einfluss auf (vii) (Cholinesterasehemmer) und (i) Calciumantagonisten bzw. NMDA-Antagonisten zwar nachweisbare, aber dennoch nicht befriedigende Therapieresultate erbringt. Dies immer vor dem Hintergrund erheblicher Nebenwirkungen in der Therapie.

Mit der Kombination von chemisch-synthetischen Arzneistoffen ist bisher das Ziel einer Heilung und auch einer Linderung auf Dauer nicht zu erreichen, da diese Substanzen jeweils auf ein einzelnes Pharmakophor zielen, und selbst bei allermodernsten Strategien, Monosubstanzen mit eingebautem Kombinationseffekt zu synthetisieren, d. h. 2 Pharmakophoren in einem Molekül zu vereinigen, die Beschränkung auf 2 Pharmakophore kaum zu überwinden sein wird und zudem jede Menge Dosierungs-, Wirksamkeits- und Verträglichkeitsfragen entstehen.

Die Entwicklung von synthetischen Wirkstoffkombinationen wird aus Gründen der Philosophie der Arzneimittelhersteller und der Zulassungsinstitutionen, und aus Gründen der Kosten beim Nachweis der Wirksamkeit wohl nicht stattfinden, da die Arzneimittelgesetzgebung für chemisch-synthetische Wirkstoffe für Kombinationen den Nachweis der Überlegenheit der Kombination gegenüber der jeweiligen Monosubstanz fordert; dies ist bei 3 Kombinationspartnern bereits extrem aufwendig. Mithin kann mit einem gut oder zumindest befriedigend wirksamen medikamentösen Therapieansatz von dieser Seite nicht gerechnet werden.

Anders sieht die Situation im Bereich der pflanzlichen Wirkstoffe aus. Zubereitungen aus Arznei- oder Nutzpflanzen sind bereits Stoffgemische mit einem breiteren Wirkstoffspektrum, und die rationale Kombination mehrerer solcher Zubereitungen kann ein breites Spektrum von Effekten abdecken. Damit wird die Wahrscheinlichkeit für das Ansprechen des Patienten auf die Medikation maximiert und eine wirksame und verträgliche, darüber hinaus auch bezahlbare Behandlungsmöglichkeit bereitgestellt.

Die WO 2007/057310 beschreibt die Verwendung von Rooibusch-Extrakten zum Schutz der Haarfarbe. Nicht detailliert offenbart ist in dieser internationalen Patentanmeldung, wie die Rooibusch-Extrakte hergestellt werden. Es bleibt offen, ob die Extrakte mit Wasser und/oder Alkoholen hergestellt werden.

Die südafrikanische Patentanmeldung 2003/3674 beschreibt Zusammensetzungen, die Extrakte enthalten, die entweder von aus fermentiertem und/oder aus unfermentiertem Rooibusch (*Aspalathus linearis*) gewonnen wurden. Die dort beschriebenen Extrakte sollen antioxidativ wirken und schädigende freie Radikale inaktivieren.

Eine Aufgabe der Erfindung ist es daher, Kombinationen von Extrakten aus verschiedenen Pflanzen zur Behandlung und/oder zur Vorbeugung dieser Krankheiten zur Verfügung zu stellen. Insbesondere sollen diese Wirkstoffe oder Zusammensetzungen keine oder nur vernachlässigbare Nebenwirkungen aufweisen, um sie auch wirksam als Vorbeugungsmittel einsetzen zu können.

Diese Aufgabe wird durch den Gegenstand der Patentansprüche gelöst.

Die vorliegende Erfindung betrifft eine Kombination von Extrakten aus verschiedenen Pflanzen, die eine Kombination von einem aus unfermentiertem Rotbusch erhaltenen Extrakt zusammen mit wenigstens zwei weiteren Extrakten, erhalten aus fermentiertem H Rotbusch, Ginseng, Grüntee und/oder Curcuma enthält. Die erfindungsgemäße Kombination ist vorzugsweise eine Kombination aus Trockenextrakten. Die Extrakte weisen vorzugsweise einen Feuchtigkeitsgehalt von < 5 Gew.-%, bevorzugter von < 4 Gew.-%, am bevorzugtesten von < 2 Gew.-% auf. Dabei enthält die Kombination Extrakte aus den folgenden Arznei- bzw. Nahrungsmittelpflanzen:

(1) grüner Rotbusch/ nicht fermentierter Rotbusch - nicht fermentierte Blätter und Zweigspitzen von *Aspalathus linearis* (BURM. F.) R. DAHLGREN und

(2)

a) Grüntee - nicht fermentierte Teeblätter von *Camellia sinensis* (L.) KUNTZE; und/oder

b) Rotbusch - fermentierte Blätter und Zweigspitzen von *Aspalathus linearis* (BURM. F.) R. DAHLGREEN, und/oder

c) Ginseng - Wurzel von *Panax ginseng* C. A. MEYER und/oder

d) Gelbwurz - Wurzel von *Curcuma longa* L. als coffeinfreie Alternative zu Grüntee

Eine zwingend vorhandene Komponente der erfindungsgemäßen Kombination von Pflanzenextrakten ist ein Extrakt aus unfermentiertem Rotbusch, der wenigstens 0,05 Gew.-%, bevorzugt wenigstens 0,1 Gew.-%, noch bevorzugter wenigstens 0,18 Gew.-%, besonders bevorzugt 0,4 Gew.-% und ganz besonders bevorzugt wenigstens 1 Gew.-% einer Verbindung der Formel I

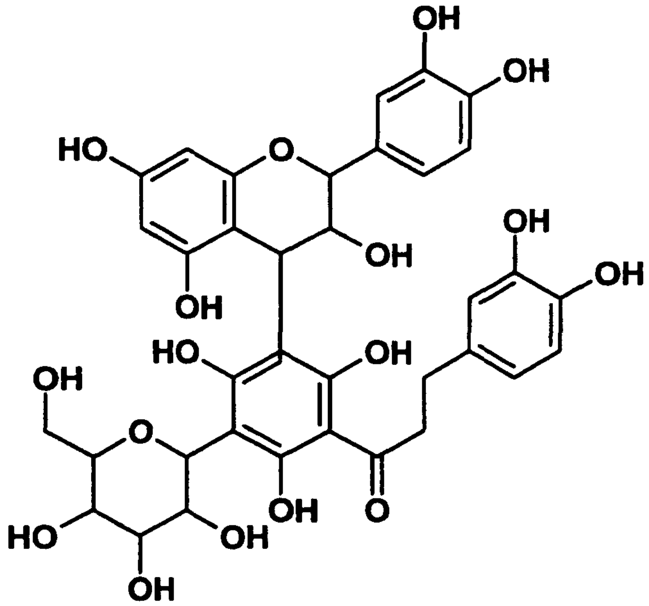

sowie deren pharmazeutisch annehmbare Salze, Derivate und Ester enthält. Die Verbindung der Formel I wird im folgenden auch kurz als Aspacat bezeichnet. Als bevorzugte Salze kommen die Kalium-, Natrium-, Ammonium- oder Gluconat-Salze in Frage. Bevorzugt sind die Ester der Essigsäure, Ameisensäure oder Propionsäure. Besonders bevorzugt sind die Ester von Fettsäuren wie $C_{10}$ bis $C_{18}$ Fettsäuren, die gegebenenfalls auch ein, zwei oder drei Doppelbindungen aufweisen können. Die Ester von Fettsäuren weisen hydrophobe Reste auf, die das Lipophilie-Verhältnis dieser Ester beeinflussen. Bevorzugte Derivate sind hierbei Kopplungsprodukte der Verbindung gemäß Formel I an Ferulasäure, Chinasäure, Kaffeesäure, Gluconsäure oder Chlorogensäure. Bevorzugt wird die Verbindung der Formel I in ihrer natürlichen Form oder als pharmazeutisch verträgliches Salz eingesetzt, weiter bevorzugt in ihrer natürlichen Form gemäß Formel I.

Unter den bevorzugten Estern sind Ameisensäure-, Essigsäure-, Propionsäure-, Glutarsäure-, Weinsäure- oder Bernsteinsäureester zu nennen. Bevorzugte Salze sind die Salze mit kationischen, organischen oder anorganischen Gegenionen zu nennen, insbesondere Alkalimetallsalze, Erdalkalimetallsalze, Ammoniumsalze oder auch Salze mit pharmazeutisch annehmbaren Säuren wie Succinate, Citrate, Tartrate.

Besonders bevorzugt eingesetzt wird ein Rotbuschextrakt aus unfermentiertem Rotbusch, mit einem Gehalt an der Verbindung gemäß Formel I von mindestens 1 Gew.-%, bevorzugt mindestens 1,5 Gew.-%, besonders bevorzugt mindestens 2,0 Gew.-%, besonders bevorzugt und ganz besonders bevorzugt mindestens 5 Gew.-%.

Der erfindungsgemäß eingesetzte Rotbuschextrakt wird dadurch hergestellt, dass

- getrocknete und zerkleinerte, unfermentierte Rotbusch-Rohware mit einem Extraktionsmittel bestehend aus Alkohol und/oder Wasser für eine vorbestimmte Extraktionsdauer bei einer Temperatur von bis zu 90°C, insbesondere bis zu 60°C extrahiert wird,
- der Extrakt filtriert und anschließend unter reduziertem Druck zur Trockne eingeengt wird.

Der erfindungsgemäß eingesetzte Extrakt aus unfermentiertem Rotbusch wird bevorzugt so hergestellt, dass als Ausgangsdroge besonders schnell und schonend getrocknete Blätter und Zweigspitzen von Aspalathus linearis

(nicht fermentiert) = "grüner" Rotbusch mit einem möglichst geringen Restfeuchte-Gehalt (< 5 %) verwendet werden.

Diese Droge wird mit Wasser oder Alkohol-Wasser-Mischungen mit wenigstens 40 % (Vol./Vol.) Wasser extrahiert. Bevorzugt eingesetzt werden Mischungen aus 10-60 %, vorzugsweise 10-50 %, vorzugsweise 15-40%, bevorzugter 15-25 % und besonders bevorzugt 20 % Methanol oder 25-60 %, bevorzugt 30-50 %, besonders bevorzugt 30 % (Vol./Vol.) Ethanol eingesetzt. Der Rest der Mischung ist Wasser.

Dadurch ist ein Extrakt mit einem Gehalt an Verbindung der Formel I von 0,05 bis 0,4 in Abhängigkeit von der eingesetzten Charge der Droge von bis zu 2,5-5 Gew.-% erzielbar. Der Gesamtflavonoidgehalt (Summe Aspalathin-artige + Rutosid-artige + Vitexin-artige ohne Verbindung gemäß Formel I) liegt bei 17-30 % mit einem Verhältnis Vitexin-artige (=C-Glycoside) zu Rutosid-artigen: </= 1,6, bevorzugt bei ≤ 2,0 und einem Gehalt an Aspalathin-artige: 14-25%.

Der Extrakt wird bevorzugt gewonnen durch ein optimiertes Extraktionsmittel, nämlich 20 % Methanol oder 30 % Ethanol in Wasser. Alternativ kann zuerst der reine Alkohol mit der Droge versetzt werden und nach einer Einweichphase von mind. 30 Minuten die entsprechende Wassermenge zugegeben werden.

Dieses Extraktionsmittel ist optimiert auf die möglichst vollständige Extraktion der Verbindung der Formel I und gleichzeitig hohe Ausbeute an Gesamtflavonoiden.

Der Extrakt unterscheidet sich von den bisher handelsüblichen Extrakten für die innerliche Anwendung in Tee-Getränken (rein wässrige Extraktion) durch höhere Gehalte an der Verbindung der Formel I; von dem Extrakt für die äußerliche Anwendung in der Kosmetik (80 % Ethanolisch; Fa. Rapps) durch das Verhältnis der Flavonoidgruppen zueinander. Der Ethanol-80%-Extrakt wurde auf einen möglichst hohen Aspalathin-Gehalt optimiert. Das Verhältnis der 3 Flavonoidgruppen (Aspalathin-artige, Rutosid-artige und Vitexin-artige = C-Glycoside) wird durch die relativ lipophile Extraktion gegenüber dem Ausgangszustand in der Droge verändert. Erkennbar wird dies besonders deutlich am Verhältnis der Vitexin-artigen zu den Rutosid-artigen Flavonoiden.

Bei dem erfindungsgemäßen Extrakt wird angestrebt, dass die 3 Flavonoidgruppen möglichst in dem selben Verhältnis im Extrakt erhalten werden, wie sie in der AusgangsDroge vorliegen (unter Berücksichtigung der natürlichen Schwankungsbreite der Droge, Verfahrensunterschiede und Unterschiede von Charge zu Charge). Dieses Verhältnis ist dann dem Verhältnis in den üblichen aus grünem Rotbusch hoher Qualität (geringer Fermentierungsgrad) hergestellten Tee-Getränken vergleichbar.

Ein Extrakt mit einem noch höheren Gehalt an Verbindung der Formel I und Gesamtflavonoiden kann dadurch gewonnen werden, dass aus dem vorher beschrieben Extrakt durch weitere Aufreinigung mit einer Größenausschlußchromatographie (Sephadex (oder ähnlich) -Säule) ein hoher Anteil der Substanz erhalten wird. Durch einen Chromatographieschritt mit Sephadex ist ein Extrakt erzielbar, der aufweist:

Verbindung der Formel I-Gehalt: > 3 %, bevorzugt > 5 %.
Gesamt-Flavonoid-Gehalt (Summe Aspalathin-artige + Rutosid-artige + Vitexin-artige ohne Verbindung gemäß Formel I): > 17 %, bevorzugt > 35 %
Verhältnis Vitexin-artige (=C-Glycoside) zu Rutosid-artigen: </= 1,6, bevorzugt </= 2,0 Gehalt Aspalathin-artige: > 20 %, bevorzugt >25 %.

Der Extrakt aus nicht fermentiertem Rotbusch ist reich an:

C-Glykosylflavone, wie Vitexin, iso-Vitexin, Orientin, Isoorientin;
Flavonol-3-0-Glykoside, wie Rutosid, Quercetin, Quercitrin, Isoquercitrin sowie Chalkone, wie Aspalathin, Nothofagin.

Typischerweise können mit dem bevorzugten Extraktionsverfahren Extrakte erhalten werden, die in Tabelle 1 zusammengefasst sind.

Die mit dem erfindungsgemäß bevorzugten Extraktionsverfahren erhaltenen Extrakte weisen vor allem Verbindungen auf, die bestimmten Gruppen zugeordnet werden können. Hierbei handelt es sich um:

a) Substanz der Formel I (Aspacat);
b) die Gruppe der Chalcone, insbesondere Quercetin, Aspalathin und Nothofagin, wobei das Aspalathin bis zu etwa 90 Gew.-% dieser Gruppe (in Tabelle 1 als Substanz Gruppe A bezeichnet) aufweisen kann;
c) die Rutosid-Gruppe umfasst Flavonoide, die Quercetin als Bestandteil enthalten. Flavonoide, die einen Zucker über eine C-O-C-Verknüpfung enthalten (Quercetin ist da Aglycon von Rutin); sowie
d) Verbindungen der sogenannten Vitexin-Gruppe. Hierunter werden Flavonoide verstanden, die einen Zucker über eine C-C-Verknüpfung enthalten. Vitexin ist das Aglycon von Apigenin, z.B. Vitexin (Apigenin-8-C-glucosid), Isovitexin (Apigenin-6-C-glucosid), Orientin (Luteolin-8-C-glucosid), Isoorientin (Luteolin-6-C-glucosid).

**Tabelle 1**. Gehalt an Flavonoiden - Substanz der Formel I, Rutosid-Gruppe, Substanz A und Vitexin -Gruppe bezogen auf die eingesetzte Droge und bezogen auf die unterschiedlich hergestellten Extrakte (ausgehend von der Droge CH G310807SA). Die % Gehalte stellen jeweils Mittelwerte aus mindestens 2 Extraktaufarbeitungen dar. (A) Die Extraktion wurde zunächst 10 Minuten mit dem reinen Alkohol durchgeführt, anschließend wurde Wasser zugegeben, bis der angegebene Alkoholgehalt eingestellt war. (B) Die Extraktion wurde von Anfang bis Ende mit einem Alkohol/Wasser-Gemisch mit dem angegebenen Alkoholgehalt durchgeführt.

| | | Gehalt [%] | | | |
|---|---|---|---|---|---|
| | Substanz I | Rutosid-Gruppe | SubstanzA-Gruppe | Vitexin-Gruppe | Droge/ Extraktverhältnis |
| **Droge (G310807SA)** | **1.09** | **0.56** | **5.81** | **0.87** | |
| Extrakt (A): Methanol 20% | 2.12 (95% 1.9 fach) | 1.85 (230% 3.3 fach) | 13.53 133% 2.3 fach) | 2.52 (190% 2.9 fach) | 3.2:1 |
| Extrakt (A): Ethanol 30% | 2.66 (144% 2.4 fach) | 2.03 (262% 3.6 fach) | 13.51 (132% 2.3 fach) | 2.14 (145% 2.5 fach) | 3.2:1 |
| Extrakt (B): Methanol 20% | 2.57 (136% 2.4 fach) | 2.45 (338% 4.4 fach) | 15.65 (169% 2.7 fach) | 3.14 (261% 3.6 fach) | 3.4:1 |
| Extrakt (B): Ethanol 30% | 3.02 (177% 2.8 fach) | 2.26 (304% 4.0 fach) | 14.51 (149.7% 2.5 fach) | 2.85 (227% 3.3 fach) | 3.2:1 |

In Klammern ist jeweils eingefügt, um wie viel % bzw. um das wie viel Fache das entsprechende Flavonoid im Vergleich zur Droge in dem jeweiligen Extrakt enthalten ist.

Die Erfindung betrifft auch die Verwendung der Kombination der Pflanzenextrakte als Medikament oder Nahrungsergänzungsmittel. Bevorzugterweise enthält das Nahrungsergänzungsmittel oder Medikament den Rotbuschextrakt in einer Menge von mindestens 25 mg, weiter bevorzugt von mindestens 50 mg, noch weiter bevorzugt von mindestens 75 mg pro Dosierungseinheit des Medikaments.

In einer bevorzugten Ausführungsform der Erfindung wird die Kombination mit dem Rotbuschextrakt mit dem erhöhten Anteil an der Verbindung gemäß Formel I zur Behandlung von neurologischen und psychiatrischen Störungen des zentralen Nervensystems, bevorzugt zur Behandlung von Demenzen, Morbus Parkinson, Depressionen und Schmerzzuständen, weiter bevorzugt Alzheimerschen Erkrankung, verwendet.

Das Verfahren zur Isolierung der chemischen Verbindung ist in Beispiel 1 ausführlich beschrieben, die Strukturformel ist in Formel I angegeben. Die Verbindung der Formel I weist sowohl Ähnlichkeiten zur Struktur des Aspalathins als auch des Catechin(4$\alpha$->2)-phloroglucinols (Figur 1) auf. Im Vergleich zu den bislang bekannten Flavonoiden weist die neue Verbindung gemäß Formel I ein hohes Molekulargewicht von 740,66 g/mol auf. Derartig hochmolekulare Naturstoffe werden üblicherweise nicht für das Wirkstoffscreening verwendet, da diese aufgrund ihres Molekulargewichts die Blut-Hirn-Schranke schlecht passieren können. Für den Wirkort Gehirn bzw. zur Behandlung von Krankheiten des zentralen Nervensystems werden derartige Stoffe daher eher als ungeeignet angesehen.

Unerwarteterweise zeigte jedoch eine "bias"-freie Untersuchung im Tele-Stereo-EEG (Elektroenzephalografie) der Ratte eine ausgeprägte, zentralnervöse, pharmakologische Aktivität der Verbindung gemäß Formel I nach oraler Verabreichung. Die pharmakologischen Untersuchungen zeigten überraschenderweise, dass die Aktivität im Modell Tele-Stereo-EEG bei Ratten dosisabhängige Veränderungen der EEG-Frequenzen bewirkt, wie sie nach Gabe klassischer Medikamente zur Behandlung von Demenzen (beispielsweise Galantamin oder Tacrin), Morbus Parkinson (L-DOPA), sowie Schmerzzuständen (beispielsweise Nefopam) bekannt sind.

Die pharmakologische Aktivität der erfindungsgemäßen Verbindung gemäß Formel I wurde im Rahmen der vorliegenden Erfindung mit den bekannten Inhaltsstoffen des Rotbuschextrakts wie Aspalathin, Catechin oder (-)-Epicatechin verglichen. Die experimentellen Untersuchungen zeigen unerwarteterweise, dass die Wirkung der Verbindung gemäß Formel I nicht mit etwa äquimolaren Mengen an Aspalathin, Catechin oder (-)-Epicatechin erreicht werden kann, obwohl die Verbindung gemäß Formel I strukturelle Ähnlichkeiten zu diesen Naturstoffen aufweist. Die neue erfindungsgemäße Verbindung gemäß Formel I besitzt eine höhere pharmakologische Aktivität als diese bekannten Inhaltsstoffe des Rotbuschextrakts und ist daher besonders geeignet für die Verwendung als Medikament.

Die Isolierung dieser neuen Verbindung mit vorteilhaften pharmakologischen Aktivitäten ermöglicht insbesondere die Herstellung eines Medikaments auf Basis der Verbindung gemäß Formel I in Kombination mit weiteren Wirkstoffen. Diese weiteren Wirkstoffe, nämlich die anderen Pflanzenextrakte, haben das Krankheitsbild auf andere Weise günstig beeinflussende Eigenschaften. Hierfür verantwortlich ist auch die Kombination dieser Verbindung mit anderen in Rotbusch enthaltenen Flavonoiden und Inhaltsstoffen im Gesamtverbund. Insbesondere sind hier Inhaltsstoffe geeignet, die eine antioxidative Wirkung aufweisen.

Insbesondere sind hier Inhaltsstoffe geeignet, die eine antioxidative und/oder neuroprotektive und/oder Nervenfunktion-aktivierende (Nervenreiz-Übertragung aktivierende) und/oder die kognitive Leistung verbessernde und/oder die Gedächtnisleistung verbessernde Wirkung aufweisen.

Die klassischen Mittel gegen Demenz, wie Tacrin und Galactamin, sorgen primär für eine Verbesserung der Nervenreizübertragung, haben aber z.B. keine neuroprotektive Wirkung. Die antioxidative Wirkung ist nur ein Aspekt der beabsichtigten Wirkbreite in dieser Kombination. Das Besondere am grünen Rotbusch ist die kognitive Verbesserung und die Memory Verbesserung. Der grüne Rotbusch schon wirkt synergistisch mit den anderen Komponenten. Grüntee bewirkt z.B. eine Verhinderung der Plaquebildung von Proteinen an den Nervenzellen und ist darüber auch neuroprotektiv. Ginseng fördert eine Neubildung von Nervenfasern. Die Kombination aller dieser Wirkungen ist ein besonderer Aspekt der Erfindung. Hinzu kommt, dass unterschiedliche Stoffgruppen ausgewählt wurden, um damit die Möglichkeit zu eröffnen, über unterschiedliche Bioverfügbarkeiten in den Geweben und unterschiedliche Wirkmechanismen möglichst gute Effekte zu erzielen.

Eine Diskriminanzanalyse der in vivo-Daten der Verbindung gemäß Formel I zeigte, wie oben erwähnt, eine Verwandtschaft zu den Medikamenten zur Behandlung von Demenzen, Morbus Parkinson, Depression und Schmerzzuständen. Da diese Medikamente ein breites Nebenwirkungsspektrum aufweisen, ist die Verwendung eines Rotbuschextrakts mit der Verbindung gemäß Formel I als Medikament vorteilhaft, da Naturstoffe üblicherweise eine geringere Nebenwirkungsrate erwarten lassen. Unerwarteterweise überschreitet die neue Substanz offensichtlich zudem die Blut-Hirnschranke. Dies ist nicht allgemein üblich für Flavonoide.

Des weiteren wird ein Herstellungsverfahren für Rotbuschextrakte sowie für die hier identifizierte pharmakologisch wirksame Verbindung zur Verfügung gestellt. Durch das erfindungsgemäße Verfahren ist es möglich, besonders geeignete Pflanzenextrakte zur Behandlung der vorstehend genannten Krankheiten zur Verfügung zu stellen.

Der erfindungsgemäß hergestellte Rotbuschextrakt weist einen Gehalt an der Verbindung gemäß Formel I (≙ Aspacat) von mindestens 0,18 Gew.-%, bevorzugt mindestens 0,4 Gew.-%, weiter bevorzugt mindestens 1,5 Gew.-%, noch weiter bevorzugt mindestens 2 Gew.-% und am meisten bevorzugt mindestens 2,5 Gew.-%, auf.

In einer besonders bevorzugten Ausführungsform wird ein Rotbuschextrakt erfindungsgemäß hergestellt, der einen Gehalt an der Verbindung gemäß Formel I von wenigstens 10, besonders bevorzugt wenigstens 20 Gew.-% aufweist.

In einer weiteren besonderen Ausführungsform der erfindungsgemäßen Kombination ist der Gesamtflavonoidgehalt im unfermentierten Rotbuschextrakt mindestens 17 Gew.-%.

Ein erfindungsgemäßes Verfahren zur Herstellung der Verbindung gemäß Formel I weist folgende Schritte auf (siehe auch Beispiel 1):

- Bereitstellen getrockneter und zerkleinerter, unfermentierter Rotbusch-Rohware,
- Extrahieren der bereitgestellten Rohware mit einem Extraktionsmittel bestehend aus einer Mischung aus einem Alkohol, bevorzugterweise Methanol und/oder Ethanol sowie Wasser für eine vorbestimmte Extraktionsdauer bei einer Temperatur von bis zu 90°C, bevorzugt bis zu 60°C,
- Filtrieren des Extrakts,
- Einengen des filtrierten Extrakts unter reduziertem Druck.

Hieran anschließen kann sich das weitere Reinigen des Extrakts in bis zu drei Schritten, nämlich

- grobe Reinigung durch Chromatographie an einer Sephadex LH20-Säule
- Feinreinigung durch Chromatographie an einer weiteren Sephadex LH20-Säule
- Auftrennung an Kieselgel, bevorzugt einer lipophilen c18-HPLC-Säule.

Wenn Präparate mit erhöhtem Gehalt an Verbindungen gemäß Formel I gewünscht sind, kann der Extrakt auch nur mit einer Chromatographiesäule aufgetrennt werden.

Bevorzugterweise ist der Feuchtegehalt der bereitgestellten Rotbusch-Rohware 4 bis 5 % oder kleiner, da auf diese Weise eine Autofermentation des Ausgangsmaterials verhindert wird. Die Rotbusch-Rohware wird bevorzugterweise alsbald der Extraktion unterzogen und nicht über längere Zeit gelagert.

Als Extraktionsmittel wird gemäß einer bevorzugten Ausgestaltung des erfindungsgemäßen Verfahrens eine Alkohol/Wasser-Mischung im Verhältnis 20:80 bis 50:50 verwendet. Bevorzugt werden Alkohole, wie Methanol, Ethanol,

Propanol oder Propan-2-ol eingesetzt). Bevorzugterweise wird eine Methanol/Wasser-Mischung von 50:50 verwendet, wenn ein besonders hoher Anteil an der Verbindung der Formel I gewünscht wird. Bei dieser bevorzugten Ausführungsform des erfindungsgemäßen Verfahrens beträgt das Verhältnis von Rohware zu Extraktionsmittel vorzugsweise ca. 1: 6, und der Schritt des Extrahierens erfolgt vorzugsweise bei erhöhter Temperatur (über 40°C) 1 Stunde lang, ist aber auch bei Raumtemperatur und einer Dauer von beispielsweise 2 bis 5 Stunden möglich. Bei niedrigem Alkoholgehalt von 20 bis 30 % (Vol./Vol.) wird ein höherer Gehalt an Flavonoiden erhalten.

Das Einengen des filtrierten Extrakts erfolgt vorzugsweise bei einem Druck von weniger als 300 mbar. Die Temperatur beträgt bei dem Einengungsschritt vorzugsweise maximal 40°C.

Die Herstellung eines erfindungsgemäßen Rotbuschextrakts erfolgt nach dem vorstehend beschriebenen Verfahren, wobei der Rotbuschextrakt nach dem Einengen bis zur Trockne (ohne nachfolgende chromatographische Reinigung) erhalten wird.

Das Messverfahren zur Bestimmung des Gehalts des Rotbuschextrakts an der Verbindung gemäß Formel I wird in Beispiel 4 ausführlich beschrieben.

Die Verbindung gemäß Formel I, deren pharmazeutisch annehmbare Salze, Derivate und Ester, sowie der erfindungsgemäße Rotbuschextrakt sind insbesondere zur Vorbeugung und/oder Behandlung von Krankheiten des zentralen Nervensystems, bevorzugt Demenzen, Morbus Parkinson, Depressionen, Schmerzzuständen und als Zellschutz-Antioxidans bzw. "Radikalfänger" geeignet. Die Behandlung von Morbus Alzheimer ist besonders bevorzugt. Der erfindungsgemäße Extrakt dient der Verbesserung der Denk-/Gedächtnisleistung, auch wenn eine Erkrankung/Demenz (noch nicht) erkennbar ist.

Erfindungsgemäß werden die Rotbuschextrakte mit der Verbindung gemäß Formel I sowohl direkt als solche oder auch als deren Ester oder Derivat in Kombination mit weiteren Wirkstoffen eingesetzt. Geeignete Derivate, Salze, Komplexe und Ester sowie deren Herstellung sind dem Fachmann bekannt. Die Herstellung pharmazeutisch annehmbarer Salze ist dem Fachmann ebenfalls bekannt. Als Salzbildner kommen alle üblichen pharmazeutisch annehmbare Säuren bzw. Anionen in Frage. Des weiteren sind Kopplungen an Säuren wie beispielsweise Ferulasäure, Chinasäure, Kaffeesäure, Gluconsäure, Chlorogensäure und verwandte Verbindungen möglich. Insbesondere ist eine Kopplung an Gluconsäure bevorzugt. Die Kopplungsprodukte der Verbindung gemäß Formel I an die vorstehenden Säuren werden als pharmazeutisch verträgliche Derivate bezeichnet. Bevorzugterweise wird die Verbindung aber als Molekül gemäß Formel I verwendet.

Der erfindungsgemäße Rotbuschextrakt kann auf an sich bekannte Weise zu Arzneimitteln und/oder zu Nahrungsergänzungsmitteln mit gesundheitsfördernden Eigenschaften verarbeitet werden. Der erfindungsgemäße Rotbuschextrakt in Kombination mit anderen Pflanzenextrakten kann z.B. in Form von Tabletten, Kapseln, Pillen, Dragees, Granulaten, Pulvern, Lutschpastillen und flüssigen Darreichungsformen wie z.B. Getränken oder löslichem Tee-Extrakt formuliert werden. Bevorzugt ist auch die Verwendung in Nahrungsergänzungsmitteln.

Bevorzugterweise werden die Nahrungsergänzungsmittel bzw. Arzneimittel oral verabreicht, wobei auch beispielsweise die topische, parenterale, intravenöse, intramuskuläre, subkutane, nasale, inhalative, rektale oder transdermale Applikation möglich ist.

Der erfindungsgemäße Rotbuschextrakt sowie die erfindungsgemäßen Arzneimittel bzw. Nahrungsergänzungsmittel können zusätzlich bevorzugt weitere Wirkstoffe, die die Wirkung des Rotbuschextrakts oder der erfindungsgemäßen Verbindung gemäß Formel I bzw. deren Salze verstärken oder die bei den benannten Krankheiten auftretenden Symptome oder Zustände ergänzend positiv beeinflussen (z.B.: weitere Radikalfänger, verschiedene enzymhemmende Stoffe, Vitamine, Lecithine, omega-3-Fettsäuren und Stoffe, die die Funktionen des Gehirns positiv beeinflussen), enthalten, sowie Ascorbinsäure.

Die erfindungsgemäße Kombination enthält neben dem Rotbuschextrakt aus unfermentierter Droge auch wenigstens eine, bevorzugt wenigstens zwei und besonders bevorzugt wenigstens drei Extrakte aus anderen Pflanzen, wobei sich insbesondere die jeweilige Menge bezogen auf eine Dosiseinheit in folgenden Bereichen bewegt:

Grüner Rotbusch-Extrakt: 10-2000 mg, bevorzugt 100-600 mg, noch bevorzugter 200-400 mg, besonders bevorzugt 50-150 mg, ganz besonders bevorzugt etwa 75 mg

Grüntee-Extrakt: 50-2000 mg, bevorzugt 50-500, bevorzugt 100-200, noch bevorzugter 75-200 mg, besonders bevorzugt etwa 100 mg

fermentierter Rotbusch-Extrakt: 10-2000 mg, bevorzugt 100-600 mg, noch bevorzugter 200-400 mg, besonders bevorzugt 50-100 mg, ganz besonders bevorzugt etwa 75 mg

Ginseng-Extrakt: 10-1000 mg, bevorzugt 50-300 mg und besonders bevorzugt 50-100 mg

Vitamin C als stabilisierender Zusatz und als Mittel zur Verbesserung der Bioverfügbarkeit: 20-1000 mg, vorzugsweise 20-500 mg, bevorzugter 20-100 mg.

Die erfindungsgemäße Kombination weist immer einen Trockenextrakt aus nicht fermentiertem ("grünem") Rotbusch (Aspalathus linearis) enthaltend Aspacat, Verbindung der Formel I, wie vorstehend beschrieben auf. Dieser

Extrakt enthält ebenso wie die Ausgangsdroge folgende Hauptflavonoide:

Aspalathin, Nothofagin, Rutosid, Quercitrin, Isoquercitrin, Quercetin, Vitexin, Orientin, Iso-Orientin.

In Abhängigkeit von dem Grad der Anreicherung kann sich der Gehalt und das Verhältnis der anderen Flavonoide ändern. Stark an Aspacat angereicherte Extrakte enthalten zwar noch weitere Flavonoide des Rotbusch, aber deutlich weniger und nicht mehr in den ursprünglichen Mengenverhältnissen.

Bevorzugt ist im Extrakt das Verhältnis der Flavonoide zueinander in etwa gleich dem in der Droge. Messbar ist dies in dem Verhältnis/Faktor, der sich ergibt, wenn man den Gehalt aller Flavonoide mit einem UV-Spektrum ähnlich Orientin/Vitexin (C-Glykoside) teilt durch den Gehalt aller Flavonoide mit einem Rutosid ähnlichen UV-Spektrum (Quercetin-Derivate). Kriterium: Dieser Faktor ist meist </= 1,6 oder </= 2,0.

Kombiniert mit dem Trockenextrakt aus grünem Rotbusch können folgende Pflanzenextrakte werden:

1) Trockenextrakt aus Rotbusch (fermentiert, Aspalathus linearis)

Auszugsmittel sind Wasser und Mischungen von Wasser mit Alkohol, wobei Alkohol eingesetzt werden kann aus: Ethanol, Methanol, Propanol-1, Propanol-2. Bevorzugt eingesetzt werden Ethanol oder Methanol in einer Menge von 0-30%, Rest: Wasser. Bei den Inhaltsstoffen sollte Aspalathin in einer Menge von > 0,4 %, bevorzugt > 3,5 % vorhanden sein.

Der Extrakt enthält ebenso wie die Ausgangsdroge folgende Flavonoide:

Aspalathin, Rutosid, Quercitrin, Isoquercitrin, Quercetin, Vitexin, Orientin, Iso-Orientin, Eriodyctyol-Glycoside.

2) Trockenextrakt aus Grüntee (Camellia sinensis)

Auszugsmittel: Wasser und Mischungen von Wasser mit Alkohol. Die Nachbehandlung des Primären Extraktes (Flüssig oder fest) mit einem Keton (Ethylacetat, Aceton, Methyl-Ethyl-Keton) ist möglich. Als Alkohol kann eingesetzt werden: Ethanol, Methanol, Propanol-1, Propanol-2, wobei bevorzugt sind: Ethanol oder Methanol 0-90 %, bevorzugt 40-85 %, Rest Wasser.
Besonders bevorzugt ist 80 % Ethanol. Erhalten werden dabei coffeinhaltige Extrakte. Falls als Ausgangsmaterial entkoffeinierte Grünteeblätter eingesetzt werden, so werden coffeinfreie Extrakte erhalten.
Besondere Inhaltsstoffe sind:

Catechine: Epigallocatechingallat (EGCG), Epicatechingallat, Catechin, Gallocatechin, Epigallocatechin, u.a.
Theanin, Coffein, Theophyllin, Theobromin.

Besondere Inhaltsstoffe:

EGCG: >10% bevorzugt: >25% (ber. als EGCG)
Gesamtcatechine (ber. als Catechin/Epicatechin): > 15% bevorzugt: >40%
Theanin: > 0,1% bevorzugt: > 1 %

Coffeinhaltige Extrakte:

Coffein: >2% bevorzugt: > 5%

3) Extrakt aus Curcuma (Curcuma longa und/oder Curcuma xanthorrhiza oder Mischungen aus beiden)

Auszugsmittel: Mischungen von Wasser mit Alkohol und oder Aceton, reine Alkohole oder eines der folgenden Ketone: Ethylacetat, Aceton, Methyl-Ethyl-Keton, sowie überkritisches Kohlendioxid.
Die Nachbehandlung des Primären Extraktes (Flüssig oder fest) mit einem Keton (Ethylacetat, Aceton, Methyl-Ethyl-Keton) ist möglich. Als Alkohol ist verwendbar: Ethanol, Methanol, Propanol-1, Propanol-2.
Bevorzugt sind: Ethanol 96-100 %, Aceton.
Besondere Inhaltsstoffe:

Curcuminoide: Curcumin, Desmethoxy-Curcumin, Bisdesmethoxy-Curcumin und optional Ätherisches Öl.
Gehalte im Extrakt: Gesamt-Curcuminoide: >1 %, bevorzugt >10 % besonders bevorzugt: >25 % Curcuminoide.

4) Trockenextrakt aus Ginseng (Panax ginseng). Ein bevorzugtes Auszugsmittel ist eine Ethanol-Wasser-Mischung mit 40 % Ethanol.

Auszugsmittel: Wasser und Mischungen von Wasser mit Alkohol.
Grundsätzlich können folgende Alkohole eingesetzt werden: Ethanol, Methanol, Propanol-1, Propanol-2.

Bevorzugt sind: Ethanol oder Methanol 5-96 %.
Besonders bevorzugt: Ethanol 40-80 %

Besondere Inhaltsstoffe:

Triterpensaponine (Ginsenoside) 2 Gruppen:
Protopanaxadiole: Ginsenosid Rb1, Rb2, Rc, Rd u.a.
Protopanaxatriole: Ginsenosid Rg1, Rg2, Rf, u.a.

Gehalt im Extrakt:

Gesamtginsenoside (HPLC ber. als Rb1): > 1% bevorzugt >2% besonders bevorzugt: >5%

Des weiteren können pharmazeutisch annehmbare Hilfs- und Trägerstoffe verwendet werden. Geeignete Hilfsstoffe sind dem Fachmann bekannt und umfassen beispielsweise Füllstoffe, Sprengmittel, Gleitmittel, Bindemittel, Benetzungsmittel, etc.

Geeignete Gleitmittel sind z.B. Silikat, Talk, Stearinsäure, Magnesium- oder Calciumstearat und/oder Polyethylenglykole.

Als Bindemittel können beispielsweise Stärken, Gummi arabicum, Gelatine, Methylcellulose, Carboxymethylcellulose oder Polyvinylpyrrolidon verwendet werden.

Aufschlussmittel sind beispielsweise Stärke, Alginsäure, Alginate oder Natriumstärkeglykolate, aufschäumende Mischungen.

Als Benetzungsmittel können beispielsweise Lecithin, Polysorbate oder Laurylsulfate verwendet werden.

Des weiteren können auch Farbstoffe und Süßungsmittel in den Formulierungen enthalten sein.

Die pharmazeutischen Zubereitungen können in bekannter Weise hergestellt werden, z.B. mittels Mischen, Granulieren, Tablettieren, Zuckerbeschichtungs- oder Überzugsbeschichtungsverfahren.

Die flüssigen Dispersionen und/oder Lösungen zur oralen Verabreichung können z.B. Getränke, Tropfen, Sirupe, Emulsionen und Suspensionen sein.

Der Sirup kann als Träger z.B. Saccharose oder Saccharose mit Glycerin und/oder Mannitol und/oder Sorbitol enthalten.

Die Suspensionen und die Emulsionen können als Träger z.B. ein natürliches Harz, Agar, Natriumalginat, Pektin, Methylcellulose, Carboxymethylcellulose oder Polyvinylalkohol enthalten.

Die Suspensionen oder Lösungen für intramuskuläre Injektionen können zusammen mit dem Wirkstoff einen pharmazeutisch annehmbare Träger, z.B. steriles Wasser, Olivenöl, Ethyloleat, Glykole, z.B. Propylenglykol, und, falls gewünscht, eine geeignete Menge an Lidocain-Hydrochlorid enthalten.

Die Lösungen zur intravenösen Injektion oder Infusion können als Träger z.B. steriles Wasser enthalten oder sie können bevorzugt in Form von sterilen, wässrigen, isotonischen Salzlösungen vorliegen.

Die Suppositorien können zusammen mit dem Wirkstoff einen pharmazeutisch annehmbare Träger, z.B. Kakaobutter, Polyethylenglykol, einen Polyoxyethylensorbitol-Fettsäureester oder Lecithin enthalten.

Zusammensetzungen für die topische Applikation, z.B. Cremes, Lotionen oder Pasten, können durch Mischen des Wirkstoffs mit einem herkömmlichen ölhaltigen oder emulgierenden Träger hergestellt werden.

Die erfindungsgemäße Kombination kann einen unfermentierten Rotbuschextrakt sowie die erfindungsgemäße Verbindung gemäß Formel I bzw. deren Salze, Derivate und Ester können in üblichen Mengen in den Nahrungsergänzungs- bzw. Arzneimitteln enthalten. In Lösung werden bevorzugterweise 0,001 bis 10 Gew.-% der erfindungsgemäßen Verbindung gemäß Formel I bzw. deren Salze, weiter bevorzugt 0,1 bis 7 Gew.-% und besonders bevorzugt 1 bis 5 Gew.-%, eingesetzt. In einer besonderen Ausführungsform werden in Lösung 0,02 bis 1 Gew.-% der erfindungsgemäßen Verbindung gemäß Formel I bzw. deren Salze eingesetzt.

Erfindungsgemäß wird der unfermentierte Rotbuschextrakt bevorzugt in Mengen eingesetzt, die einer Menge an der Verbindung gemäß Formel I von 1 bis 1000 mg, weiter bevorzugt 10 bis 600 mg, noch weiter bevorzugt 50 bis 400 mg und am meisten bevorzugt 50 bis 250 mg entsprechen.

Wenn ein Rotbuschextrakt eingesetzt wird, der einen sehr hohen Anteil an der Verbindung gemäß Formel I aufweist, kann ein derartiger Extrakt bei Arzneimitteln bei Nahrungsergänzungsmitteln in einer Menge eingesetzt werden,

die zwischen 3 und 600 mg, bevorzugt zwischen 5 und 100 mg und besonders bevorzugt zwischen 10 und 50 mg pro Tagesdosis liegt.

Die oben beschriebenen Nahrungsergänzungsmittel (Lebensmittel) bzw. Arzneimittel können auf herkömmliche Methoden hergestellt und in einer pharmazeutisch geeigneten Form verabreicht werden.

Die erfindungsgemäß bevorzugten, festen Nahrungsergänzungs- bzw. Arzneimittel können zusätzlich bevorzugt 1 bis 50 Gew.-%, stärker bevorzugt 1 bis 20 Gew.-%, besonders bevorzugt 1 bis 10 Gew.-% Füllstoffe enthalten.

Als Füllstoffe können eine oder mehrere Verbindungen verwendet werden, die einen Teil des Materials zum Erreichen der erforderlichen und gewünschten Tabletten- oder Kapselmasse liefern. Einsetzbar sind unter anderem mikrokristalline Cellulose in verschiedenen Partikelgrößen, insbesondere mit einer mittleren Partikelgröße im Bereich von 20 μm bis 200 μm, insbesondere im Bereich von 50 μm bis 150 μm, wie z.B. etwa 100 μm, wie die bekannten Avicel-Produkte wie Avicel PH-101 und PH-102. Weitere geeignete Füllstoffe sind z.B. Maisstärke, Kartoffelstärke, Laktose, Cellaktose (eine Mischung aus Cellulose und Laktose), Calciumphosphat, Dextrose, Mannit, Maltodextrin, Isomalt, gegebenenfalls auch Sorbit und Saccharose. Falls eine Direktverpressung vorgesehen ist, sollte bei der Auswahl der Füllstoffe darauf geachtet werden, dass Qualitäten verwendet werden, die für die Direktverpressung von Tabletten geeignet sind. Dies wird bei den kommerziellen Produkten jeweils vom Hersteller angegeben bzw. kann durch einfache Versuche überprüft werden. Das am stärksten bevorzugte Füllmittel ist mikrokristalline Cellulose (Handelsprodukte sind beispielsweise Avicel, Vivapur und Emcocel).

Geeignete Sprengmittel sind im Stand der Technik bekannt. Sprengmittel werden häufig auch mit dem englischen Begriff "Disintegrants" bezeichnet. Erfindungsgemäß bevorzugte Sprengmittel sind z.B. Crospovidone (Kollidon CL) und Stärke bzw. vorverkleisterte Stärke, insbesondere das Handelsprodukt "Starch 1500". Weitere geeignete Stärken sind z.B. unter den Bezeichnungen Lycatab PGS, Prejel und Sepistab ST 200 kommerziell erhältlich. Weiterhin können auch die bekannten sogenannten "Super Disintegrants" verwendet werden, wie Croscarmellose Natrium (z.B. Ac-Di-Sol, u.a.) und Carboxymethylstärke Natrium (z.B. Explotab, Primojel, u.a.). Besonders bevorzugt sind Stärken wie Starch 1500.

Der Gehalt an Sprengmittel ist in der Regel 1 bis 25 Gew.-%, vorzugsweise 1 bis 20 Gew.-%, insbesondere 2 bis 15 Gew.-%. Geeignete Bereiche für den Gehalt an Sprengmittel sind auch z.B. 2 bis 5 Gew.-% oder 15 bis 20 Gew.-%, in Abhängigkeit der verwendeten Sprengmittel, Füllstoffe und übrigen Zusatzstoffe.

Erfindungsgemäß kann die Zusammensetzung als Gleitmittel eine oder mehrere Verbindungen enthalten, die die Herstellung und die Verarbeitung der Tablette unterstützen. Verwendbare Gleitmittel sind unter anderem Stearinsäure und deren Derivate, wie Calciumstearat, und insbesondere Natriumstearylfumarat (das z.B. unter der Bezeichnung Pruv kommerziell erhältlich ist) und Magnesiumstearat, Glycerolmono-, -di-und insbesondere -tristearat, hydriertes Pflanzenöl (z.B. Lubritab, Dynasan, Sterotext) oder ein Polyethylenglykol (z.B. Lutrol, Carbowax).

Der Gehalt an Gleitmittel ist in der Regel 0,1 bis 4 Gew.-%, vorzugsweise 0,2 bis 4 Gew.-%.

Optional kann die erfindungsgemäße pharmazeutische Zusammensetzung ein oder mehrere Fließregulierungsmittel umfassen. Geeignete Fließregulierungsmittel sind Magnesiumtrisilikat, Talk und insbesondere Siliziumdioxid (z.B. Aerosil). Falls die Zusammensetzung ein Fließregulierungsmittel umfasst, ist dieses in der Regel in einer Menge von 0,5 bis 5 Gew.-%, vorzugsweise 1 bis 4 Gew.-%, insbesondere 2 bis 3 Gew.-% vorhanden.

Die erfindungsgemäßen pharmazeutischen Zusammensetzungen können ebenfalls noch Stabilisatoren für den Wirkstoff enthalten, wie Ascorbinsäure, Zitronensäure, Weinsäure, Milchsäure, etc., bevorzugt Ascorbinsäure und/ oder Zitronensäure. Der Gehalt an Stabilisator (falls vorhanden) ist in der Regel im Bereich von 0,1-10 Gew.-%, bevorzugt 0,5 bis 10 Gew.-%, bevorzugt 1 bis 3 Gew.-%.

Die erfindungsgemäßen pharmazeutischen Zusammensetzungen können weitere übliche pharmazeutisch verträgliche Zusatz- und Hilfsstoffe enthalten, bevorzugt enthalten sie aber außer den vorstehend angegebenen (Füllstoff, Sprengmittel, Schmiermittel und gegebenenfalls Fließregulierungsmittel und Stabilisator) keine weiteren Hilfsstoffe.

Einige Füllstoffe wie mikrokristalline Cellulose können auch als Bindemittel dienen. Auch Füllstoffe mit Bindemittelfunktion zählen daher im Rahmen dieser Anmeldung zu den Füllstoffen.

Liegt die erfindungsgemäße pharmazeutische Zusammensetzung als Tablette vor, kann sie mit einem oder mehreren Beschichtungsmitteln film beschichtet sein. Verwendbare Beschichtungsmittel sind Schellack oder Schellack-Mischungen, Hypromellose (Hydroxypropylmethylcellulose), Polyvinylalkohol, Natriumcarboxymethylcellulose und verschiedene Methacrylsäurepolymere (Eudragite), wobei Hypromellose und insbesondere Eudragite, Schellack oder Schellack-Mischungen bevorzugt sind. Das Beschichten der Tabletten erfolgt auf übliche Art und Weise. In der Beschichtung können außer dem Beschichtungsmittel weitere übliche Bestandteile von Tablettenbeschichtungen wie Weichmacher, Pigmente, Porenbildner oder Suspensionsstabilisatoren vorhanden sein wie z.B. Polyethylenglykol (PEG), Talk oder Titandioxid und gegebenenfalls auch Laktose.

Das Tablettengewicht ist nicht besonders eingeschränkt, üblich sind Tabletten mit 100 mg bis 500 mg bei Verwendung von reinen Wirkstoffen und 500 mg bis 1500 mg bei Verwendung von Extrakten und Pflanzenpulvern. In Kapseln werden Mengen von 100 mg bis 1000 mg verwendet.

Die Dosierungseinheit des Arzneimittels oder Nahrungsergänzungsmittels kann beispielsweise enthalten:

- bei peroralen Arzneiformen:

  bevorzugt 1 bis 1000 mg, bevorzugter 40 bis 800 mg, besonders bevorzugt 150 bis 500 mg, noch bevorzugter 300 bis 600 mg, Rotbuschextrakt pro Tagesdosis. Bei Verwendung der Verbindung gemäß Formel I, sowie deren pharmazeutisch annehmbaren Salze, Derivate und Ester werden 1/100 bis 1/20 der vorstehenden Mengen eingesetzt.
  Die Tagesdosis kann beispielsweise in 1 bis 3 Einzeldosen, vorzugsweise in zwei Einzeldosen, täglich verabreicht werden. Es kann auch vorgesehen sein, dass 1-10 Einzeldosen Rotbuschextrakt enthaltend die Verbindung gemäß Formel 1 täglich verabreicht werden.

- bei parenteralen Arzneiformen (zum Beispiel intravenös, subkutan, intramuskulär): bevorzugt 3 bis 60 mg, besonders bevorzugt 10 bis 30 mg, Wirkstoff pro Tagesdosis.
  Die Tagesdosis kann beispielsweise in 1 bis 3 Einzeldosen, vorzugsweise in einer Einzeldosis, täglich verabreicht werden.
- bei Arzneiformen zur rektalen Applikation:

  bevorzugt 40 bis 80 mg, besonders bevorzugt 60 mg, Wirkstoff gemäß Formel I der Extrakt pro Tagesdosis.

  Die Tagesdosis kann beispielsweise in 1 bis 3 Einzeldosen, vorzugsweise in einer Einzeldosis, täglich verabreicht werden.
- bei Arzneiformen zur Applikation auf die Haut und Schleimhäute (z.B. Lösungen, Lotionen, Emulsionen, Salben usw.):

  bevorzugt 40 bis 80 mg Wirkstoff, besonders bevorzugt 60 mg, Wirkstoff pro Einzeldosis. Bezieht man den Gehalt an Verbindung gemäß Formel I auf die fertige Lösung, Lotion, Emulsion oder Salbe, dann beträgt der Gewichtsanteil bezogen auf derartige salbenartige Arzneimittel zwischen 0,05 und 20 Gew.-%, bevorzugt zwischen 0,2 und 1 Gew.-% an Verbindung der Formel I bezogen auf die cremeartige Präparation.

  Die Tagesdosis kann beispielsweise in 1 bis 6 Einzeldosen, vorzugsweise in 1 bis 3 Einzeldosen, täglich verabreicht werden.

Es können 10-2000 mg, vorzugsweise 10-1000 mg, vorzugsweise 10-500 mg erfindungsgemäße Kombination pro Dosiseinheit eingesetzt werden.

**Beschreibung der Figuren**

**Figur 1** zeigt die Strukturformel von Aspalathin (1) und Catechin(4$\alpha$->2)-phloroglucinol (2).

**Figur 2** zeigt die Nummerierung der Atome in der Verbindung gemäß Formel I.

**Figur 3** zeigt ein UV-Spektrum der Verbindung mit der Formel I.

**Figur 4** zeigt ein UV-Spektrum von Aspalathin.

**Figur 5** zeigt ein UV-Spektrum von Rutosid.

**Figur 6** zeigt ein UV-Spektrum von Orientin.

**Figur 7** zeigt ein UV-Spektrum von Homoorientin.

**Figur 8** zeigt ein UV-Spektrum von Vitexin.

**Beispiel 1 -** Isolierung der Verbindung gemäß Formel I

Zur Herstellung aus der Droge wird unfermentierter grüner Rotbusch vom Hersteller Rooibos Ltd. Clanwilliam Süd-Afrika verwendet.

Als Ausgangsmaterial werden schonend auf einen Feuchtegehalt von kleiner als 10% (besser kleiner als 4%) getrocknete, unfermentierte und zerkleinerte Blätter und/oder Zweigspitzen von Aspalathus linearis verwendet.

Dieses Rohmaterial wird mit einer Mischung aus Methanol und Wasser im Verhältnis 50:50 (Volumenteile) bei 60°C für 1 Stunde lang unter Rotation extrahiert, wobei das Verhältnis Rohware:Lösemittel 1:7 beträgt. Danach wird die Flüssigkeit von den Pflanzenteilen abfiltriert und die Pflanzenteile erneut auf die gleiche Weise extrahiert und filtriert.

Die beiden Filtrate werden vereinigt und bei vermindertem Druck (220 mbar) und 55°C vom Methanol befreit. Die verbleibende wässrige Lösung wird mit Wasser zum 5-fachen des Gewichts der eingesetzten Menge getrockneter Pflanzenteile verdünnt und einer Flüssig-flüssig-Verteilung unterzogen.

3 Liter der wässrigen Lösung werden 4 mal mit jeweils 1,5 L wassergesättigtem n-Butanol ausgeschüttelt und die vereinigten Butanolphasen unter reduziertem Druck zur Trockne gebracht. Die Ausbeute beträgt ca. 10% der eingesetzten Menge getrockneter Pflanzenteile.

Anschließend erfolgt zunächst eine Grob- und danach eine Feintrennung des Butanolextrakts.

Grobtrennung:

Ca. 50 g Butanolextrakt werden über eine Sephadex LH20-Säule (6 cm Innendurchmesser und 80 cm Füllhöhe = 2260 ml Sephadex LH20) mit 50 Vol% Methanol chromatographiert. Dazu werden die 50 g Butanolextrakt in 400 ml mobiler Phase gelöst und auf die Trennsäule gegeben. Die Säule wird mit einem Fluss von 1,8 ml/Min. so lange gewaschen, bis 3 L Eluat abgetropft sind. Die Säulenfüllung wird nach dem vollständigen Abtropfen der restlichen mobilen Phase entnommen und in 3 L 100%-igem Methanol 10 Minuten lang ausgerührt (extrahiert). Die stationäre Phase wird abfiltriert und das Eluat getrocknet. Der Rückstand beträgt ca. 0,5 bis 1 % der eingesetzten Menge Pflanzenteile = Methanolextrakt.

Erste Feintrennung

Ca. 4 g Methanolextrakt werden über eine Sephadex LH20-Säule (3,5 cm Innendurchmesser und 50 cm Füllhöhe = 480 ml Sephadex LH20) mit 80 Vol% Methanol chromatographiert. Dazu werden die 4 g Butanolextrakt in 40 ml mobiler Phase gelöst und auf die Trennsäule gegeben. Die Säule wird mit einem Fluss von 2,4 ml/Min. betrieben und Fraktionen von je 10 Min. Dauer = 24 ml Eluat aufgefangen. Die gesuchte Substanz befindet sich in den Fraktionen Nr. 48-65. Die Ausbeute beträgt bei 4 g Methanolextrakt ca. 0,5 bis 1 g.

Zweite Feintrennung:

Trennsäule: 250 x 30 mm
Stationäre Phase: Reprosil C18 Aqua 10 μm
Mobile Phase: Methanol 35% (V/V)
Fluss: 1,5 ml/Min.
Fraktionsgröße: 10 Min. = 15 ml
Substanz I befindet sich in den Fraktionen 41 bis 52.

Die vereinigten Fraktionen 41 bis 52 werden lyophilisiert.
Ausbeute ca. 125 mg Substanz I aus 4 g Methanolextrakt.
Chromatographische Reinheit ca. 97% (HPLC).

**Beispiel 2 -** Strukturaufklärung der Verbindung gemäß Formel I

Die durch säulenchromatographische Auftrennung erhaltene Verbindung wurde mit verschiedenen Methoden charakterisiert.

Figur 2 zeigt die Nummerierung der Atome in der erfindungsgemäßen Verbindung gemäß Formel I.

**Beispiel 3 -** Messverfahren zur Bestimmung des Gehalts an Verbindung gemäß Formel I

Der Gehalt der Verbindung nach Formel I in Rotbusch und Zubereitungen aus Rotbusch (Tee, Extrakt, Tabletten) wird mittels HPLC/DAD nach der Methode des externen Standards bestimmt. Zur Gehaltsbestimmung wird die Substanz der Verbindung gemäß

Formel I als externer Standard verwendet. Die Auswertung erfolgt bei 280 nm Detektionswellenlänge. Um Oxidationsvorgänge in der Analyselösung zu verhindern, wird den Proben Askorbinsäure zugesetzt.

Das bevorzugt verwendete HPLC-Gerät ist Acquity UPLC/Alliance 2695; Detektor: DAD, 200 bis 400 nm; Säule: Reprosil-Pur ODS-3, 125 x 3 mm, 3 μm, Fa. Dr. Maisch; Säulentemperatur: 60°C.

Eluent: Eluent A: Wasser/Ameisensäure 100/0,2 (V/V);
Eluent B: Acetonitril/Methanol/Wasser/Ameisensäure 50/25/25/0,2 (V/V/V/V).
Injektionsvolumen: 20 μL.
Laufzeit: 95 Min.
Retentionszeit der Verbindung gemäß Formel I: 39,5 Min.

Tabelle 2 (Gradient):

| Zeit (Min.) | Fluss (ml/Min.) | Eluent A % | Eluent B % | Gradientensteigun g |
|---|---|---|---|---|
| 0 | 0,3 | 100 | 0 | |
| 40 | 0,3 | 70 | 30 | linear |
| 60 | 0,5 | 20 | 80 | linear |
| 80 | 0,5 | 20 | 80 | linear |
| 81 | 0,3 | 100 | 0 | |
| 95 | 0,3 | 100 | 0 | |

Als Standardlösung wird verwendet:

1 mg der Verbindung gemäß Formel I, genau gewogen, und ca. 20 mg Askorbinsäure werden mit 2 ml Methanol gelöst und mit Wasser zu 20,00 ml aufgefüllt.
Sollkonzentration: 0,05 mg/mL der Verbindung gemäß Formel I und 1 mg/mL Askorbinsäure.

Als Analysenlösung wird verwendet:

<u>Arzneimittelformulierung:</u>

Die zu untersuchende Probe, beispielsweise eine Tablette, wird in einer Pulvermühle pulverisiert und über einem Sieb mit Maschenweite 250 μm gesiebt.
Ca. 0,5 g pulverisierte Probe werden zusammen mit ca. 50 mg Askorbinsäure genau in einen 50 mL Messkolben eingewogen, mit 10 mL Methanol 40°C versetzt und für 10 Min. im Ultraschallbad bei 40°C extrahiert. Anschließend wird mit Wasser bis zur Marke aufgefüllt, kräftig geschüttelt und erneut für 10 Min. im Ultraschallbad bei 40°C extrahiert. Nach dem Erkalten wird gegebenenfalls mit Wasser bis zur Marke aufgefüllt und die Lösung für 5 Min. bei 9300 g zentrifugiert. Der Überstand wird über einen 0,45 μm Membranfilter direkt in ein Braunglasfläschchen für den automatischen Probengeber der HPLC-Anlage filtriert.

<u>Trockenextrakt Auszugsmittel Wasser:</u>

Ca. 125 mg Rotbuschextrakt werden zusammen mit ca. 25 mg Askorbinsäure in einen 25 mL Messkolben gewogen, mit ca. 22 mL Wasser versetzt, kräftig geschüttelt, gegebenenfalls im Ultraschallbad behandelt und mit Wasser bis zur Marke aufgefüllt.

<u>Trockenextrakt Auszugsmittel nicht Wasser:</u>

Ca. 125 mg Rotbuschextrakt werden zusammen mit ca. 25 mg Askorbinsäure in einen 25 mL Messkolben

gewogen, mit ca. 2,5 mL Methanol versetzt und für 10 Min. im Ultraschallbad behandelt. Anschließend wird mit Wasser bis zur Marke aufgefüllt, kräftig geschüttelt und erneut für 10 Min. im Ultraschallbad behandelt.

Auswertung:

Standardlösung und Analyselösung werden direkt hintereinander unter den selben Bedingungen chromatographiert. Die UV-Spektren der Referenzsubstanz werden mit der zur selben Retentionszeit im Analysenchromatogramm detektierten Substanz verglichen und bei Übereinstimmung der Peak als Verbindung gemäß Formel I nach folgender Berechnungsformel berechnet:

$$\text{Gehalt [\%]} = \frac{\text{Peakfläche Analyse} \cdot \text{Verdünnung Analysenlösung (mL)} \cdot \text{Einwaage Standard (mg)} \cdot 100}{\text{Peakfläche Standard} \cdot \text{Verdünnung Standardlösung (mL)} \cdot \text{Einwaage Analyse (mg)}}$$

**Beispiel 4 -** Rotbuschextrakt mit hohem Gesamtflavonoidgehalt

a) Herstellverfahren

10 g getrocknete und pulverisierte, unfermentierte Rotbusch-Rohware, wird mit 0,2 g Askorbinsäure und mit 60 ml Ethanol absolut versetzt und 10 Minuten lang bei 40°C mit Ultraschall mazeriert. Danach werden 140 ml demineralisiertes Wasser zugesetzt, kräftig geschüttelt oder gerührt und dann erneut für 10 Minuten lang bei 40°C mit Ultraschall mazeriert. Danach wird das ganze zentrifugiert (ca. 9000×g), der Überstand filtriert, der Rückstand mit 100 ml 30% (V/V) versetzt und erneut 10 Minuten lang bei 40°C mit Ultraschall mazeriert. Danach wird zentrifugiert und der Überstand filtriert. Die vereinigten Filtrate werden unter reduziertem Druck (max. 300 mbar) eingeengt auf ca. 10-20 ml und dann gefriergetrocknet. Alternativ zur Gefriertrocknung ist auch die Sprühtrocknung geeignet. Im Falle der Sprühtrocknung wird das Einengen so weit betrieben, bis eine Viskosität von ca. 130 mPascal erreicht ist. Diese Lösung wird dann sprühgetrocknet. Falls erforderlich können die üblichen Hilfsstoffe (Aerosil, Lactose, Maltodextrine) der Lösung vor Sprühtrocknung zugesetzt werden.

Durch dieses Verfahren wird ein "Extrakt (A): Ethanol 30 %" gemäß Tabelle 1 erhalten.

b) Analyse

0.5 g Extrakt (genau eingewogen) wird zusammen mit ca. 50 mg Ascorbinsäure in einen 50.0 mL Messkolben eingewogen, mit 10 mL Methanol (40 °C) versetzt und für 10 min im Ultraschallbad bei 40 °C extrahiert. Anschließend wird mit Wasser bis zur Marke aufgefüllt, kräftig geschüttelt und erneut für 10 min im Ultraschallbad bei 40 °C extrahiert. Nach dem Erkalten wird gegebenenfalls mit Wasser bis zur Marke aufgefüllt und die Lösung für 5 min bei 9300×g zentrifugiert.

Vom Überstand werden 1 ml genommen, über einen Spritzenfilter in ein Vial (Braunglas) filtriert und per HPLC vermessen. Bei den Drogen wurde die restliche Lösung (49 ml) mittels Rotavapor abrotiert und der Rückstand nach Gefriertrocknung bestimmt.

4a) Substanz 1

Die Identifizierung der Substanz mit der Formel I im Chromatogramm des Extraktes 1 (G110907SA erfolgte über ein zur Verfügung stehendes Vergleichsspektrum sowie über das UV-Spektrum (Figur 3).

Der Gehalt an Substanz 1 in verschiedenen Extrakten wurde unter Verwendung des bekannten Gehaltes im Extrakt 1 (G110907SA) (0.95%) anhand folgender Formel berechnet (*F*-Fläche, *V*-Volumen, *m*-Masse, *g*-Gehalt, *Ana*-Analyse, *St*-Standard):

$$g_{Subs\tan z1}(\%) = \frac{F_{Ana}V_{Ana}}{m_{Ana}relF_{St}}100\%$$

Wobei die relative Fläche *relF* folgendermaßen berechnet wurde:

$$relF = \frac{100F_{St}}{V_{Extrakt}m_{Extrakt}0.95} = 12.52 \pm 0.04$$

4b) Flavonoide der Substanz A-Gruppe

Flavonoide der Substanz A-Gruppe sind durch UV-Maxima bei 287 nm und 228 nm charakterisiert. Alle Peaks, die eine weitgehende Übereinstimmung mit diesem Spektrum zeigen (Figur 4), werden zu dieser Gruppe gezählt und der Gehalt anhand folgender Formel berechnet:

$$g_{Flavonoi\deg ruppeA}(\%) = \frac{F_{Ana-GruppeA}V_{Ana}m_{Rutosid}}{m_{Ana}F_{Rutosid}V_{Rutosid}}k_f 100\%$$

Als Standard wird Rutosid verwendet, der Korrekturfaktor $k_f$ beträgt 0.4.

4c) Flavonoide der Rutosid-Gruppe

Flavonoide werden anhand eines Rutosid-Vergleichsspektrums (Figur 5) dieser Gruppe zugeordnet. Der Gehalt wird anhand folgender Formel berechnet:

$$g_{FlavonoideRutosid}(\%) = \frac{F_{Ana-Rutosid}V_{Ana}m_{Rutosid}}{m_{Ana}F_{Rutosid}V_{Rutosid}}100\%$$

4d) Flavonoide der Vitexin-Gruppe

Flavonoide werden anhand eines Vitexin-Vergleichsspektrums dieser Gruppe zugeordnet, zu der auch Orientin und Homoorientin gehören (Figur 6 bis Figur 8). Als Standard wird anstelle des Vitexins Homoorientin verwendet, da Vitexin (und auch Orientin) bei der Herstellung der Standardlösungen Probleme mit der Löslichkeit zeigten. Der Gehalt wird anhand folgender Formel berechnet:

$$g_{FlavonoideVitexin}(\%) = \frac{F_{Ana-Vitexin}V_{Ana}m_{Homoorientin}}{m_{Ana}F_{Homoorientin}V_{Homoorientin}}100\%$$

**Beispiel 5 -** Herstellung eines angereicherten Rotbuschextraktes mit erhöhtem Gehalt an Verbindung I:

**Durchführung der Sephadex-Säule**

750 mg eines Extraktes nach Beispiel 4 wurden in 6 ml MeOH gelöst, zentrifugiert und die überstehende Lösung auf die Säule gegeben.
500 mg gelöster Extrakt (auf die Säule)
Sephadex-LH-20-Säule: Innendurchmesser: 1,4 cm; Länge: 27 cm
Offene Säulenchromatographie: Methanol als Elutionsmittel; 65 Reagenzgläser mit durchschnittlich 2,5 ml Lösung. Tropfgeschwindigkeit: zwischen 10-17 Tropfen pro Minute.
Kontrolle der Reagenzgläser mittels DC, Bedingungen: Kieselgelplatten (Merck, Kieselgel 60 F254), Fließmittel: EtOAc: HCOOH:CH3COOH:H2O, 100:11:11:27, v:v:v:v; Detektion: Naturstoffreagenz. Die Lösungen der Reagenzgläser, die ein ähnliches Flavonoidmuster aufwiesen, wurden vereinigt, das Lösungsmittel abrotiert und der Rückstand nach Gefriertrocknung gewogen.

Erhaltene Fraktionen:

Fr. 1: (RG 21-31): 106,2 mg (flavonoidhaltig nach DC)
Fr. 2: (RG 33-46): 18,3 mg (flavonoidhaltig nach DC)

Fr. 3: (RG 47-52): 10,03 mg (flavonoidhaltig nach DC)

Die Fraktionen 1-3 sowie Mischungen und Überschneidungen daraus liefern nach dem Trocknen geeignete Extrakte.

**Beispiel 6 -** Herstellung eines angereicherten Rotbuschextraktes mit noch weiter erhöhtem Gehalt an Verbindung I:

**Weitere Aufreinigung der Fraktion 2 aus Beispiel 5 mittels Mitteldruck-LC an einer RP18-Säule.**

System: Mitteldruck (LPLC)
Säule: RP 18 silica gel (50 x 1.2 cm)
Mobile Phase: MeOH : H2O (MeOH 30% zu 100%)
Flußgeschwindigkeit: 0.7 ml/min
Detektion: 280 nm
Anzahl der aufgefangenen Fraktionen: 70 mit jeweils ca. 3 ml.
Die vereinigten Fraktionen Nr. 43-46 ergeben nach Trocknung den gewünschten Extrakt.

**Beispiel 7 -** Formulierung einer Kombination aus 4 Pflanzenextrakten zur Vorbeugung und Behandlung von Demenzerkrankungen:

Hartgelatinekapsel:

1 Kapsel enthält (mg):

| | |
|---|---|
| Grüntee Extr. | 100 |
| Rotbusch Extr. Nicht fermentiert | 75 |
| Rotbusch Extr. fermentiert | 75 |
| Ginseng Extr. | 50 |
| Askorbinsäure | 50 |
| Maltodextrin | 20 |
| Siliciumdioxid | q.s. |
| Magnesiumstearat | q.s. |

Tagesdosis für Vorbeugung: 1-4 Kapseln
Tagesdosis für die Therapie: 3-8 Kapseln

Für die folgenden Beispiele 8-16 wurden die Extrakte bezogen bei:

**Extrakte**

Im Rahmen der vorliegenden Erfindung wurden bevorzugt nachfolgend aufgeführte Extrakte eingesetzt. Alternativ können gleichwertige Extrakte verwendet werden.

**Grüntee-Extrakt:**

Fa. Frutarom Switzerland Ltd.
Rütiwiesstrasse 7
CH-8820 Wädenswil
www.frutarom.com

**Ginseng-Extrakt:**

Fa. Frutarom Switzerland Ltd.
Rütiwiesstrasse 7
CH-8820 Wädenswil
www.frutarom.com

**Rotbusch-Extrakt (fermentiert):**

Rooibos Ltd.
Rooibos Avenue
Clanwilliam 8135 RSA
Süd-Afrika

**Curcuma-Extrakt:**

Plantextrakt GmbH & Co KG
Dutendorfer Str. 5-7
91487 Vestenbergsgreuth

Als unfermentierter Rotbusch-Extrakt wurde der unfermentierte Rotbusch-Extrakt gemäß Beispiel 4 eingesetzt.

**Beispiel 8 -** Formulierungen einer Kombination aus 4 Pflanzenextrakten zur Vorbeugung und Behandlung von Demenzerkrankungen:

Hartgelatinekapsel:

| 1 Kapsel enthält (mg): | |
|---|---|
| Curcuma Extr. | 15 |
| Rotbusch Extr. Nicht fermentiert* | 100 |
| Rotbusch Extr. fermentiert | 100 |
| Ginseng Extr. | 50 |
| Askorbinsäure | 50 |
| Piperin | 25 |
| Maltodextrin | 20 |
| Siliciumdioxid | q.s. |
| Magnesiumstearat | q.s. |

Tagesdosis für Vorbeugung: 1-4 Kapseln
Tagesdosis für die Therapie: 3-8 Kapseln
* (enthält 0,4 Gew.-% der Verbindung gemäß Formel I)

**Beispiel 9 -** Formulierungen einer Kombination aus 3 Pflanzenextrakten zur Vorbeugung und Behandlung von Demenzerkrankungen:

Hartgelatinekapsel:

| 1 Kapsel enthält (mg): | |
|---|---|
| Grüntee Extr. | 150 |
| Rotbusch Extr. Nicht fermentiert* | 100 |
| Ginseng Extr. | 50 |
| Askorbinsäure | 25 |
| Maltodextrin | 50 |
| Siliciumdioxid | q.s. |
| Magnesiumstearat | q.s. |
| * (enthält 0,4 Gew.-% der Verbindung gemäß Formel I) | |
| Tagesdosis für Vorbeugung: 1-4 Kapseln | |
| Tagesdosis für die Therapie 3-8 Kapseln | |

**Beispiel 10 -** Formulierungen einer Kombination aus 3 Pflanzenextrakten zur Vorbeugung und Behandlung von Demenzerkrankungen:

Hartgelatinekapsel:

| 1 Kapsel enthält (mg): | |
|---|---|
| Grüntee Extr. | 100 |
| Rotbusch Extr. Nicht fermentiert* | 100 |
| Rotbusch Extr. fermentiert | 150 |
| Askorbinsäure | 30 |
| Maltodextrin | 0 |
| Siliciumdioxid | q.s. |
| Magnesiumstearat | q.s. |
| * (enthält 0,4 Gew.-% der Verbindung gemäß Formel I) | |
| Tagesdosis für Vorbeugung: 1-4 Kapseln | |
| Tagesdosis für die Therapie: 3-8 Kapseln | |

**Beispiel 11 -** Formulierungen einer Kombination aus 3 Pflanzenextrakten zur Vorbeugung und Behandlung von Demenzerkrankungen:

Hartgelatinekapseln:

| 1 Kapsel enthält (mg): | |
|---|---|
| Curcuma Extr. | 25 |
| Rotbusch Extr. Nicht fermentiert* | 100 |
| Rotbusch Extr. fermentiert | 200 |
| Askorbinsäure | 30 |
| Maltodextrin | 25 |
| Siliciumdioxid | q.s. |

(fortgesetzt)

| | |
|---|---|
| Magnesiumstearat | q.s. |
| * (enthält 0,8 Gew.-% der Verbindung gemäß Formel I) | |
| Tagesdosis für Vorbeugung: 1-3 Kapseln | |
| Tagesdosis für die Therapie: 2-6 Kapseln | |

Vergleich **Beispiel 12** - Formulierungen einer Kombination aus 2 Pflanzenextrakten zur Vorbeugung und Behandlung von Demenzerkrankungen:

Hartgelatinekapsel:

| 1 Kapsel enthält (mg): | |
|---|---|
| Grüntee Extr. | 200 |
| Rotbusch Extr. Nicht fermentiert* | 100 |
| Askorbinsäure | 50 |
| Maltrodextrin | 30 |
| Siliciumdioxid | q.s. |
| Magnesiumstearat | q.s. |
| * (enthält 0,4 Gew.-% der Verbindung der Formel I) | |
| Tagesdosis für Vorbeugung: 1-3 Kapseln | |
| Tagesdosis für die Therapie: 3-6 Kapseln | |

Vergleich **Beispiel 13** - Formulierungen einer Kombination aus 2 Pflanzenextrakten zur Vorbeugung und Behandlung von Demenzerkrankungen:

Hartgelatinekapsel:

| 1 Kapsel enthält (mg): | |
|---|---|
| Rotbusch Extr. nicht fermentiert | 150 |
| Rotbusch Extr. fermentiert | 200 |
| Askorbinsäure | 30 |
| Maltodextrin | 0 |
| Siliciumdioxid | q.s. |
| Magnesiumstearat | q.s. |
| Tagesdosis für Vorbeugung: 1-3 Kapseln | |
| Tagesdosis für die Therapie: 3-6 Kapseln | |

Vergleich **Beispiel 14** - Formulierungen einer Kombination aus 2 Pflanzenextrakten zur Vorbeugung und Behandlung von Demenzerkrankungen:

Hartgelatinekapsel:

| 1 Kapsel enthält (mg): | |
|---|---|
| Rotbusch Extr. nicht fermentiert | 150 |
| Grüntee-Extrakt | 200 |
| Askorbinsäure | 30 |

(fortgesetzt)

| | |
|---|---|
| Maltodextrin | 0 |
| Siliciumdioxid | q.s. |
| Magnesiumstearat | q.s. |
| Tagesdosis für Vorbeugung: 1-4 Kapseln | |
| Tagesdosis für die Therapie: 3-6 Kapseln | |

Vergleich **Beispiel 15** - Formulierungen einer Kombination aus 2 Pflanzenextrakten zur Vorbeugung und Behandlung von Demenzerkrankungen:

Hartgelatinekapsel:

| 1 Kapsel enthält (mg): | |
|---|---|
| Rotbusch Extr. nicht fermentiert | 250 |
| Ginseng-Extrakt | 75 |
| Askorbinsäure | 30 |
| Maltodextrin | 25 |
| Siliciumdioxid | q.s. |
| Magnesiumstearat | q.s. |
| Tagesdosis für Vorbeugung: 1-2 Kapseln | |
| Tagesdosis für die Therapie: 2-4 Kapseln | |

Vergleich **Beispiel 16** - Formulierungen einer Kombination aus 2 Pflanzenextrakten zur Vorbeugung und Behandlung von Demenzerkrankungen:

Hargelatinekapsel:

| 1 Kapsel enthält (mg): | |
|---|---|
| Rotbusch Extr. nicht fermentiert | 250 |
| Curcuma-Extrakt | 50 |
| Askorbinsäure | 50 |
| Maltodextrin | 35 |
| Siliciumdioxid | q.s. |
| Magnesiumstearat | q.s. |
| Tagesdosis für Vorbeugung: 1-2 Kapseln | |
| Tagesdosis für die Therapie: 2-4 Kapseln | |

**Beispiel 17** - Herstellung einer Hartgelatinekapsel

| | |
|---|---|
| Grüntee Extrakt | 250 mg |
| Rotbuschextrakt nicht fermentiert | 50 mg |
| Ginsengextrakt | 50 mg |
| Askorbinsäure | 25 mg |
| Maltodextrin | 25 mg |
| Siliciumdioxid und Magnesiumstearat q.s. | |

Vergleich **Beispiel 18** - Herstellung einer Hartgelatinekapsel

| | |
|---|---|
| Grüntee Extrakt (60% Epigalocatechingallat) | 150 mg |
| Rotbuschextrakt nicht fermentiert | 50 mg |
| Askorbinsäure | 25 mg |
| Maltodextrin | 25 mg |
| Siliciumdioxid und Magnesiumstearat q.s. | |

**Beispiel 19** - Herstellung einer Hartgelatinekapsel

| | |
|---|---|
| Grüntee-Extrakt | 100,0 mg |
| Rotbuschtee-Extrakt | 75,0 mg |
| Grüner Rotbuschtee-Extrakt* | 75,0 mg |
| Ginseng-Extrakt | 50,0 mg |
| Ascorbinsäure | 50,0 mg |
| Magnesiumstearat (q.s. z.B.: 0,57%) | 2,0 mg |
| Aerosil 200 (q.s. z.B.: 0,14%) | 0,5 mg |

* Gehalt an Substanz nach Formel I: 0,1%.

**Beispiel 20** - Herstellung einer Lutschpastille, die auch zu einem Teegetränk aufgelöst werden kann (=Lutschpastille/ Teepastille)

| | |
|---|---|
| Grüntee-Extrakt | 43 mg |
| Rotbuschtee-Extrakt | 32 mg |
| Grüner Rotbuschtee-Extrakt | 32 mg |
| Ginseng-Extrakt | 21 mg |
| Ascorbinsäure | 22 mg |
| Grundmasse | 1.850 mg |

| Grundmasse: | % |
|---|---|
| Arabisches Gummi | 29,0 |
| Zucker | 24,0 |
| Glucosesirup | 24,0 |
| Wasser | 22,6 |
| Aromenmischung | 0,4 |

* Gehalt an Substanz nach Formel I: 0,18 %.

**Beispiel 21** - Herstellung einer Lutschpastille, die auch zu einem Teegetränk aufgelöst werden kann (=Lutschpastille/ Teepastille)

| | |
|---|---|
| Grüntee-Extrakt | 30 mg |
| Grüner Rotbuschtee-Extrakt | 60 mg |
| Ginseng-Extrakt | 20 mg |

(fortgesetzt)

| | |
|---|---|
| Ascorbinsäure | 35 mg |
| Grundmasse | 2.015 mg |

| Grundmasse: | % |
|---|---|
| Arabisches Gummi | 30,0 |
| Zucker | 12,0 |
| Fruktose | 12,0 |
| Apfeldicksaft | 21,0 |
| Wasser | 24,4 |
| Aromenmischung | 0,6 |

* Gehalt an Substanz nach Formel I: 0,18 %.

**Beispiel 22** - Nachweis der Wirksamkeit

Es wurde eine randomisierte Doppelblindstudie am Menschen durchgeführt, um die Verbesserung der Gedächtnisleistung am Menschen durch eine grüner Rotbusch-Rotbusch-Grüntee-Ginseng-Kapsel (beschrieben in Beispiel 19) zu belegen. Als Vergleichspräparat wurde eine Placebo-Kapsel, die nur mikrokristalline Zellulose enthielt, verabreicht. Als Dosierungsschema wurden täglich 2 x 3 Hartkapseln vor dem Frühstück und vor dem Mittagessen über einen Zeitraum von vier Wochen verabreicht. Ausgewertet wurde der Test durch quantitativ-topographische EEGs in Ruhe sowie in Gegenwart von Provokationen. Darüber hinaus füllten die Probanden Fragebögen aus.

Die vorliegende Studie zeigt nach Einmalgabe der Kapseln mit Extrakt von grünem Rotbusch, Rotbusch, Grünem Tee und Ginseng niedrigere delta und alpha Leistung bei geschlossenen Augen im Vergleich zu Placebo. Bei Betrachtung der für kognitive Funktionen wichtigen fronto-temporalen Hirnregion kommt es zu statistisch signifikanten Unterschieden im Frequenzmuster. Insbesondere während der Durchführung des KLT sowie des Memory-Tests kommt es auf den kombinierten Elektrodenpositionen F7, T3, Fz, T5 zu einem verstärkten Abfall der delta und alpha1 Wellen unter Verum-Bedingungen.

Nach vierwöchiger repetitiver Gabe der Präparate konnten auch für die Gesamtheit der Elektrodenpositionen signifikante Unterschiede zwischen Placebo und Verum während der Durchführung von KLT und Memory-Test dokumentiert werden. Während bei der Durchführung des KLT wiederum die alpha und beta Wellen stärker als bei Placebo abnahmen, kam es bei Durchführung des Memory- Tests neben einem Abfall der delta Wellen - wie auch nach Einzeldosierung - zu einer statistisch signifikanten Abnahme der alpha1 Wellen. Da der Abfall der alpha und beta Wellen während der Durchführung dieser Tests mit den psychometrischen Ergebnissen korreliert und auch allgemein in der Literatur als Surrogatparameter für Gedächtnisfunktionen gilt, wird das Ergebnis im Sinne einer Verbesserung der kognitiven Leistung gewertet.

Das Ergebnis der Studie liefert erste Hinweise für eine Verbesserung der kognitiven Leistung durch Einnahme von Kapseln mit einer Mischung von Extrakten von grünem Rotbusch, Rotbusch, Grüntee und Ginseng schon nach Einzeldosierung. Dabei scheinen vor allem Gedächtnisprozesse betroffen zu sein. Auch nach repetitiver Gabe konnten statistisch auffällige Unterschiede zur Placebogabe eine Stunde nach Verabreichung dokumentiert werden, die im Sinne eines positiven Einflusses auf Gedächtnisfunktionen zu interpretieren sind.

## Patentansprüche

1. Kombination von Extrakten aus verschiedenen Pflanzen, **dadurch gekennzeichnet, dass** die Kombination einen aus unfermentiertem Rotbusch erhaltenen Extrakt zusammen mit wenigstens zwei weiteren Extrakten, erhalten aus fermentiertem Rotbusch, Ginseng, Grüntee, und/oder Curcuma enthält, wobei der Rotbuschextrakt aus unfermentiertem Rotbusch eine Verbindung der Formel I

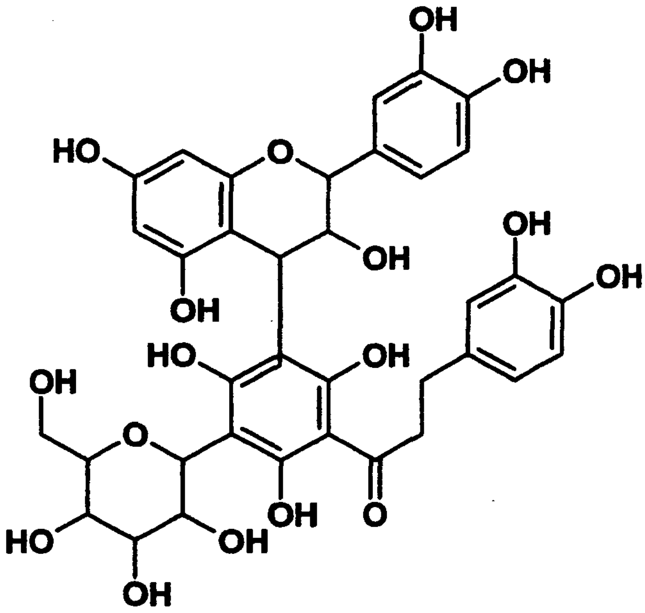

sowie deren pharmazeutisch annehmbare Salze, und Ester in einer Menge von mindestens 0,05 Gew.-% aufweist.

2. Kombination nach Anspruch 1, **dadurch gekennzeichnet, dass** der Extrakt aus unfermentiertem Rotbusch wenigstens 0,1 Gew.-% der Verbindung gemäß Formel I enthält.

3. Kombination nach Anspruch 1, **dadurch gekennzeichnet, dass** der Extrakt aus unfermentiertem Rotbusch wenigstens 0,4 Gew.-% der Verbindung der Formel I sowie deren pharmazeutisch annehmbare Salze, und Ester aufweist.

4. Kombination nach Anspruch 1, **dadurch gekennzeichnet, dass** das Präparat neben einem Trockenextrakt aus nicht fermentiertem Rotbusch gemäß einem der vorhergehenden Ansprüche wenigstens drei weitere Trockenextrakte, ausgewählt aus der Gruppe bestehend aus fermentiertem Rotbusch, Ginseng, Grüntee und/oder Curcuma aufweist.

5. Kombination nach einem der vorhergehenden Ansprüche, **dadurch gekennzeichnet, dass** der Rotbuschextrakt aus nicht fermentiertem Rotbusch dadurch hergestellt wird, dass getrocknete und zerkleinerte, unfermentierte Rotbusch-Rohware mit einem Extraktionsmittel bestehend aus einem Alkohol in einer Menge zwischen 20 und 50 % (Vol./Vol.) und Wasser für eine vorbestimmte Extraktionsdauer bei einer Temperatur von bis zu 90°C, insbesondere bis zu 60°C extrahiert wird, der Extrakt anschließend filtriert und anschließend unter reduziertem Druck zur Trockne eingeengt wird.

6. Kombination nach Anspruch 5, **dadurch gekennzeichnet, dass** der zur Trockne eingeengte Extrakt gelöst und durch Chromatographie, insbesondere Größenausschlußchromatographie weiter gereinigt wird.

7. Kombination nach einem der vorhergehenden Ansprüche 5 oder 6, **dadurch gekennzeichnet, dass** der Trockenextrakt aus nicht fermentiertem Rotbusch durch Extraktion mit einem Wasser-Alkohol-Gemisch erhalten wurde, wobei das Gemisch Ethanol und/oder Methanol in einer Menge von 20 bis 30 Gew.-% beinhaltet.

8. Kombination nach einem der Ansprüche 1 bis 7, **dadurch gekennzeichnet, dass** sie einen Trockenextrakt aus grünem Tee, hergestellt durch Extraktion mit einer Mischung von Wasser oder Wasser mit bis zu 80 % (Vol/Vol) Alkohol enthält.

9. Kombination nach einem der Ansprüche 1 bis 7, **dadurch gekennzeichnet, dass** sie einen Trockenextrakt aus fermentiertem Rotbusch, hergestellt durch Extraktion mit Wasser oder einer Mischung aus Wasser und Alkohol enthält.

10. Kombination nach einem der Ansprüche 1 bis 7, **dadurch gekennzeichnet, dass** sie einen Trockenextrakt aus Curcuma, hergestellt durch Extraktion mit Alkohol, $CO_2$ oder einer Mischung aus Wasser mit Alkohol und/oder

Aceton enthält.

11. Kombination nach einem der Ansprüche 1 bis 7, **dadurch gekennzeichnet, dass** sie einen Trockenextrakt aus Ginseng, hergestellt durch Extraktion mit einer Mischung von Wasser und insbesondere bis zu 80 % (Vol/Vol) Alkohol enthält.

12. Kombination nach einem der Ansprüche 1 bis 11, **dadurch gekennzeichnet, dass** der Gesamtflavonoidgehalt im unfermentierten Rotbuschextrakt mindestens 17 Gew.-% ist.

13. Arzneimittel, **dadurch gekennzeichnet, dass** es eine Kombination nach einem der Ansprüche 1 bis 12 enthält.

14. Arzneimittel zur Vorbeugung und/oder Behandlung von Demenzerkrankungen, **dadurch gekennzeichnet, dass** es eine Kombination nach einem der Ansprüche 1 bis 12 enthält.

15. Nahrungsergänzungsmittel, **dadurch gekennzeichnet, dass** es eine Kombination nach einem der Ansprüche 1 bis 12 enthält.

**Claims**

1. Combination of extracts of various plants, **characterised in that** the combination contains an extract obtained from unfermented rooibos together with at least two other extracts, obtained from fermented rooibos, ginseng, green tea and/or curcuma, the rooibos extract of unfermented rooibos comprising a compound of formula I

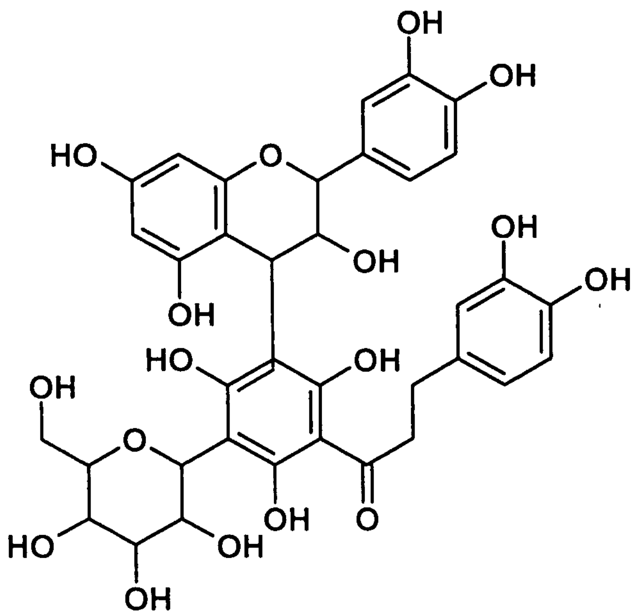

as well as the pharmaceutically acceptable salts and esters thereof in a quantity of at least 0.05 % by weight.

2. Combination according to clam 1, **characterised in that** the extract of unfermented rooibos contains at least 0.1 % by weight of the compound according to formula I.

3. Combination according to claim 1, **characterised in that** the extract of unfermented rooibos comprises at least 0.4 % by weight of the compound of formula I as well as the pharmaceutically acceptable salts and esters thereof.

4. Combination according to claim 1, **characterised in that** in addition to a dry extract of unfermented rooibos according to any one of the preceding claims, the preparation comprises at least three further dry extracts, selected from the group consisting of fermented rooibos, ginseng, green tea and/or curcuma.

5. Combination according to any one of the preceding claims, **characterised in that** the rooibos extract of unfermented

rooibos is prepared **in that** dried and crushed, unfermented rooibos raw material is extracted using an extracting agent consisting of an alcohol in a quantity of between 20 and 50 % (vol./vol.) and water for a predetermined extraction time at a temperature of up to 90°C, in particular up to 60°C, the extract is then filtered and subsequently evaporated to dryness under reduced pressure.

**6.** Combination according to claim 5, **characterised in that** the extract evaporated to dryness is dissolved and further purified by chromatography, in particular size-exclusion chromatography.

**7.** Combination according to any one of the preceding claims 5 or 6, **characterised in that** the dry extract of unfermented rooibos was obtained by extraction using a water-alcohol mixture, the mixture containing ethanol and/or methanol in a quantity of from 20 to 30 % by weight.

**8.** Combination according to any one of claims 1 to 7, **characterised in that** it contains a dry extract of green tea, prepared by extraction with a mixture of water or water with up to 80 % (vol./vol.) of alcohol.

**9.** Combination according to any one of claims 1 to 7, **characterised in that** it contains a dry extract of fermented rooibos, prepared by extraction with water or a mixture of water and alcohol.

**10.** Combination according to any one of claims 1 to 7, **characterised in that** it contains a dry extract of curcuma, prepared by extraction with alcohol, $CO_2$ or a mixture of water with alcohol and/or acetone.

**11.** Combination according to any one of claims 1 to 7, **characterised in that** it contains a dry extract of ginseng, prepared by extraction with a mixture of water and in particular up to 80 % (vol./vol.) of alcohol.

**12.** Combination according to any one of claims 1 to 11, **characterised in that** the total flavonoid content in the unfermented rooibos extract is at least 17 % by weight.

**13.** Drug, **characterised in that** it contains a combination according to any one of claims 1 to 12.

**14.** Drug for the prevention and/or treatment of dementia disorders, **characterised in that** it contains a combination according to any one of claims 1 to 12.

**15.** Food supplement, **characterised in that** it contains a combination according to any one of claims 1 to 12.

## Revendications

**1.** Combinaison d'extraits de différentes plantes, **caractérisée en ce que** la combinaison présente un extrait obtenu à partir de rooibos non fermenté et d'au moins deux autres extraits obtenus à partir de rooibos fermenté, de ginseng, de thé vert et/ou de curcuma, l'extrait de rooibos constitué de rooibos non fermenté étant un composé de formule I

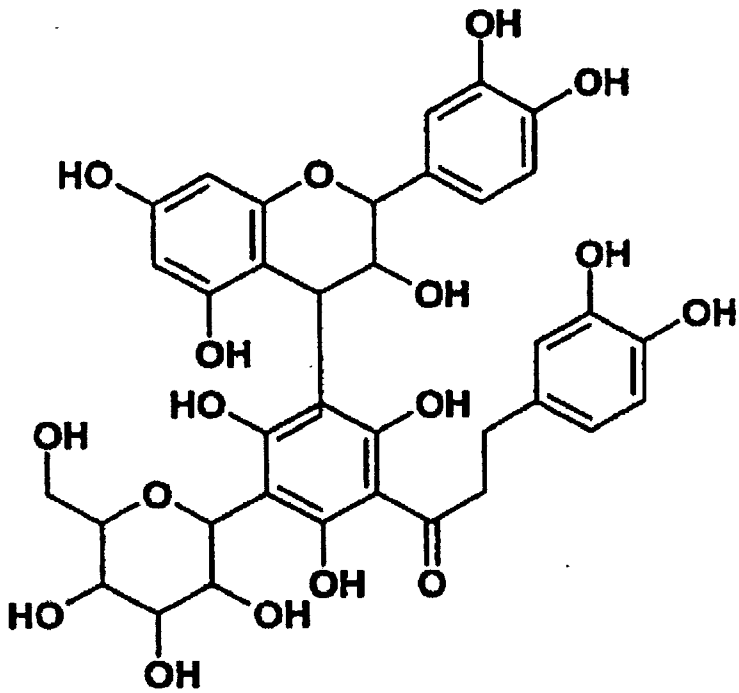

et ses sels et esters pharmaceutiquement acceptables en une quantité d'au moins 0,05% en poids.

2. Combinaison selon la revendication 1, **caractérisée en ce que** l'extrait de rooibos non fermenté contient au moins 0, 1 % en poids du composé selon la formule I.

3. Combinaison selon la revendication 1, **caractérisée en ce que** l'extrait de rooibos non fermenté présente 0,4% en poids du composé de formule I et ses sels et esters pharmaceutiquement acceptables.

4. Combinaison selon la revendication 1, **caractérisée en ce que** la préparation présente, parallèlement à un extrait sec de rooibos non fermenté selon l'une des revendications précédentes, au moins trois autres extraits secs choisis dans le groupe comprenant le rooibos fermenté, le ginseng, le thé vert et/ou le curcuma.

5. Combinaison selon l'une des revendications précédentes, **caractérisée en ce que** l'extrait de rooibos du rooibos non fermenté est produit en extrayant la matière première de rooibos séchée et concassée, non fermentée avec un agent d'extraction constitué d'un alcool en une quantité entre 20 et 50% (vol/vol) et d'eau pendant une durée d'extraction prédéterminée à une température allant jusqu'à 90°C, en particulier, jusqu'à 60°C, l'extrait étant ensuite filtré et ensuite, concentré à dessiccation sous pression réduite.

6. Combinaison selon la revendication 5, **caractérisée en ce que** l'extrait concentré à dessiccation est dissous et est encore purifié par chromatographie, en particulier, chromatographie d'exclusion.

7. Combinaison selon l'une des revendications 5 ou 6 précédentes, **caractérisé en ce que** l'extrait sec de rooibos non fermenté a été obtenu par extraction avec un mélange eau-alcool, le mélange contenant de l'éthanol et/ou du méthanol en une quantité de 20 à 30% en poids.

8. Combinaison selon l'une des revendications 1 à 7, **caractérisée en ce qu'**elle contient un extrait sec de thé vert produit par extraction avec un mélange d'eau ou d'eau comportant jusqu'à 80% (vol/vol) d'alcool.

9. Combinaison selon l'une des revendications 1 à 7, **caractérisée en ce qu'**elle contient un extrait sec de rooibos fermenté, produit par extraction avec de l'eau ou un mélange d'eau et d'alcool.

10. Combinaison selon l'une des revendications 1 à 7, **caractérisée en ce qu'**elle contient un extrait sec de curcuma, produit par extraction avec de l'alcool, du $CO_2$ ou un mélange d'eau et d'alcool et/ou d'acétone.

**11.** Combinaison selon l'une des revendications 1 à 7, **caractérisée en ce qu'**elle contient un extrait sec de ginseng produit par extraction avec un mélange d'eau et en particulier, de jusqu'à 80% (vol/vol) d'alcool.

**12.** Combinaison selon l'une des revendications 1 à 11, **caractérisée en ce que** la teneur totale en flavonoïdes dans l'extrait de rooibos non fermenté est d'au moins 17% en poids.

**13.** Médicament, **caractérisé en ce qu'**il contient une combinaison selon l'une des revendications 1 à 12.

**14.** Médicament pour la prévention et/ou le traitement de démences, **caractérisé en ce qu'**il contient une combinaison selon l'une des revendications 1 à 12.

**15.** Complément alimentaire, **caractérisé en ce qu'**il contient une combinaison selon l'une des revendications 1 à 12.

Figur 1

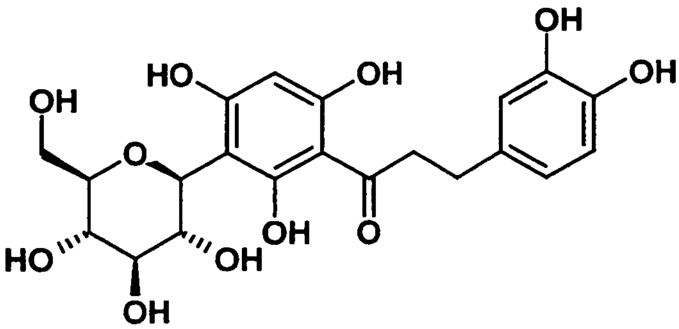

(1)

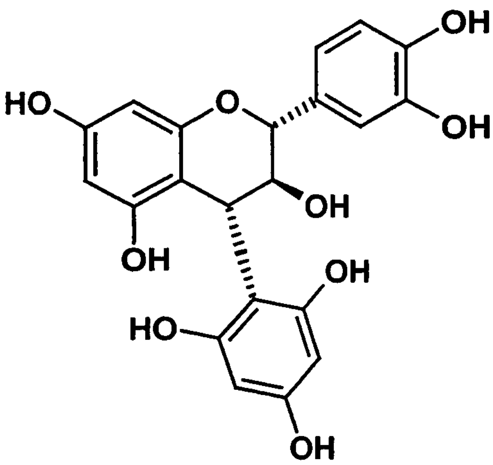

(2)

Figur 2

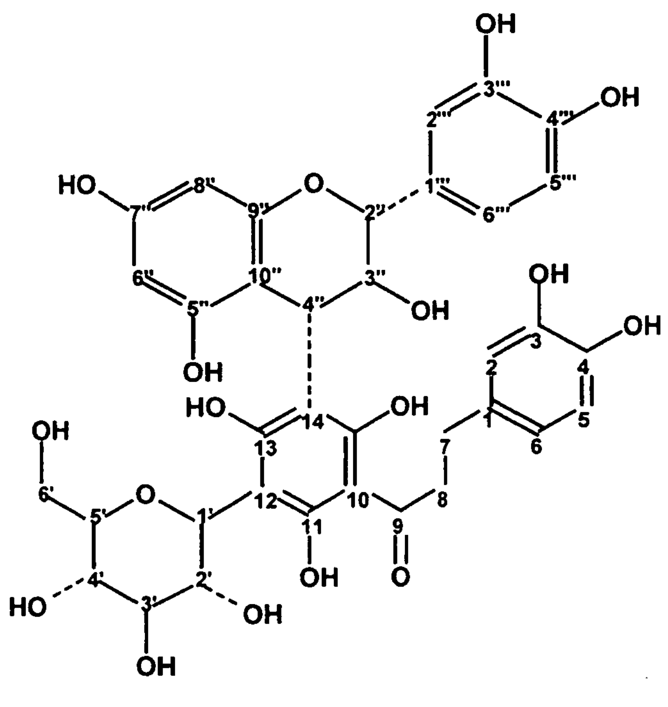

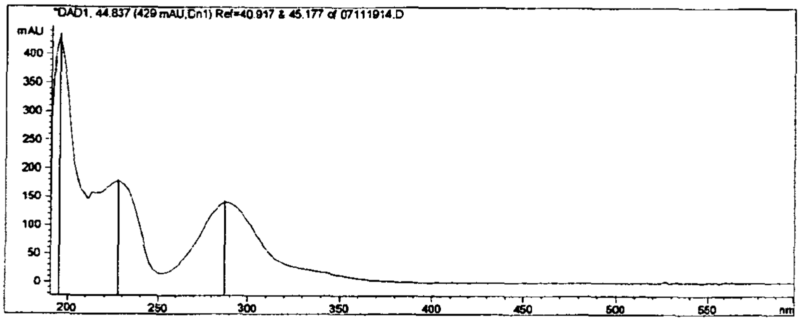


**Figur 3. UV-Spektrum von Substanz 1**

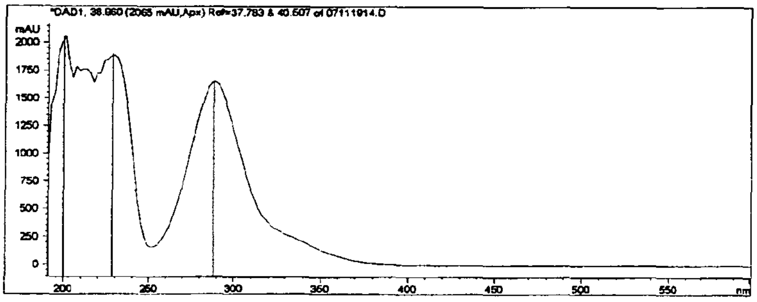


**Figur 4. UV-Spektrum von Aspalathin**

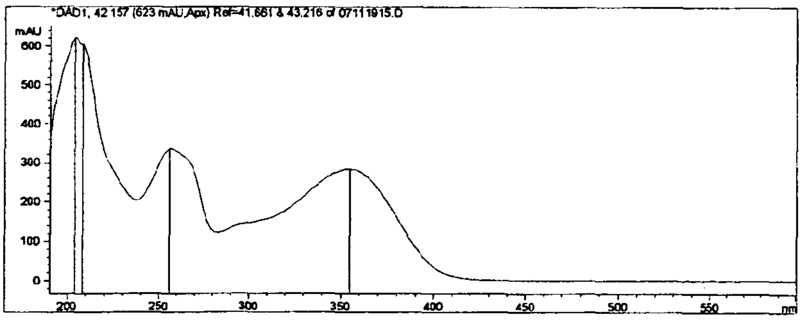


**Figur 5. UV-Spektrum von Rutosid**

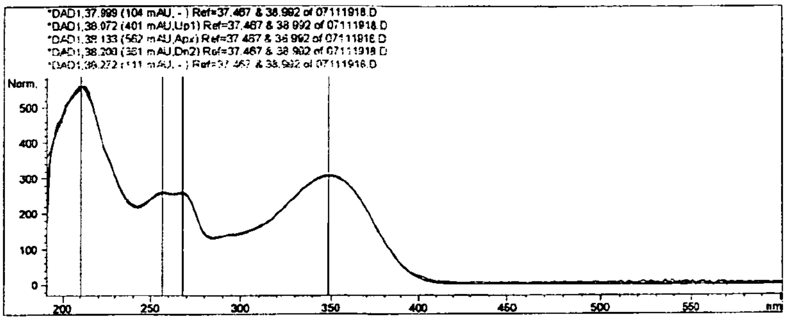


**Figur 6. UV-Spektrum von Orientin**

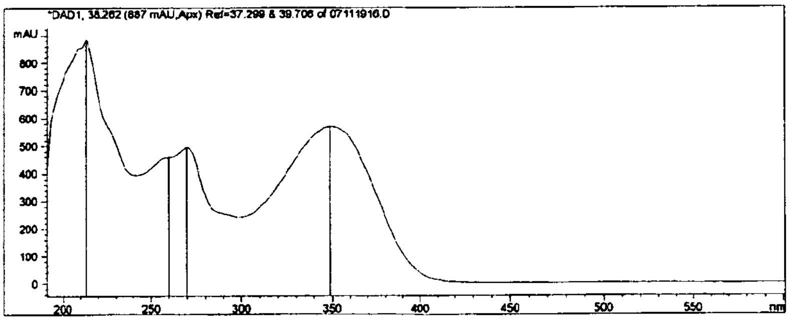


**Figur 7. UV-Spektrum von Homoorientin**

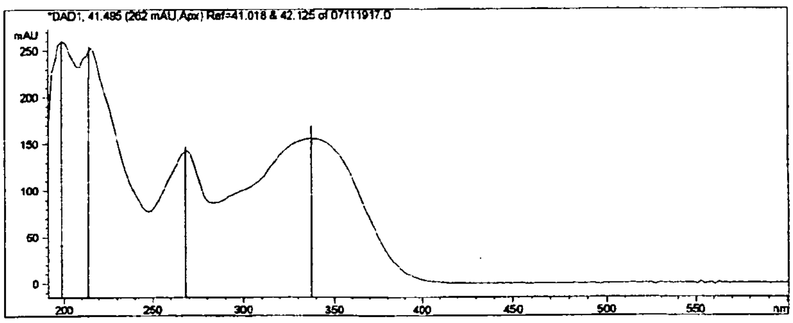


**Figur 8. UV-Spektrum Vitexin**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 102005004438
- WO 2007057310 A
- ZA 20033674


### In der Beschreibung aufgeführte Nicht-Patentliteratur


- **BRAMATI et al.** *J. Agric. Food Chem.,* 2003, vol. 51, 7472-7474
- **SCHULZ et al.** *Eur. Food Res. Technol.,* 2003, vol. 216, 539-543
- Plantextrakt, the nature network. 11. September 2005